# EUROPEAN PATENT SPECIFICATION

(11) **EP 1 523 558 B1**
(45) Date of publication and mention of the grant of the patent: **04.07.2007**
(21) Application number: 02807566.1
(22) Date of filing: 05.08.2002
(51) Int. Cl.: C12N 15/62, C12N 15/18, C12N 15/82, C07K 14/585, C07K 14/425, A01H 5/00, A01H 5/10

(54) **PRODUCTION OF PEPTIDES AND PROTEINS BY ACCUMULATION IN PLANT ENDOPLASMIC RETICULUM-DERIVED PROTEIN BODIES**
HERSTELLUNG VON PEPTIDEN UND PROTEINEN DURCH ANHÄUFUNG IN VOM ENDOPLASMATISCHEN RETICULUM ABGELEITETEN PROTEINKÖRPERN
PRODUCTION DE PEPTIDES ET DE PROTEINES PAR ACCUMULATION DANS DES CORPS PROTEIQUES DERIVES DU RETICULUM ENDOPLASMIQUE D'UNE PLANTE

(30) Priority: 28.06.2002 ES 200201508
(43) Date of publication of application: 20.04.2005
(73) Proprietor: Era Plantech S.L., 08028 Barcelona (ES)
(72) Inventor: LUDEVID MUGICA, Maria, Dolores, 08028 Barcelona (ES); TORRENT QUETGLAS, Margarita, 08023 Barcelona (ES); LASSERRE-RAMASSAMY, Sabine, 66180 Villeneuve de la Raho Roussillon (FR)
(74) Representative: Fisher, Adrian John
(86) International application number: PCT/EP2002/008716
(87) International publication number: WO 2004/003207

(56) References cited:
- WO-A-00/40738
- US-A- 5 948 682
- GELI M I ET AL: "TWO STRUCTURAL DOMAINS MEDIATE TWO SEQUENTIAL EVENTS IN GAMMA-ZEIN TARGETING: PROTEIN ENDOPLASMIC RETICULUM RETENTION AND PROTEIN BODY FORMATION" PLANT CELL, AMERICAN SOCIETY OF PLANT PHYSIOLOGISTS, ROCKVILLE, MD, US, vol. 6, no. 12, 1 December 1994 (1994-12-01), pages 1911-1922, XP002017847 ISSN: 1040-4651 cited in the application
- EMS-MCCLUNG STEPHANIE C ET AL: "Mutational analysis of the maize gamma zein C-terminal cysteine residues." PLANT SCIENCE (SHANNON), vol. 162, no. 1, January 2002 (2002-01), pages 131-141, XP002245792 ISSN: 0168-9452
- ALVAREZ INAKI ET AL: "Lysine-rich gamma-zeins are secreted in transgenic Arabidopsis plants." PLANTA (BERLIN), vol. 205, no. 3, July 1998 (1998-07), pages 420-427, XP002245793 ISSN: 0032-0935
- PHILIP REENA ET AL: "Localization of beta-glucuronidase in protein bodies of transgenic tobacco seed by fusion to an amino terminal sequence of the soybean lectin gene." PLANT SCIENCE (SHANNON), vol. 137, no. 2, 9 October 1998 (1998-10-09), pages 191-204, XP002245794 ISSN: 0168-9452
- TORRENT M ET AL: "LYSINE-RICH MODIFIED GAMMA-ZEINS ACCUMULATE IN PROTEIN BODIES OF TRANSIENTLY TRANSFORMED MAIZE ENDOSPERMS" PLANT MOLECULAR BIOLOGY, NIJHOFF PUBLISHERS, DORDRECHT, NL, vol. 34, no. 1, 1 May 1997 (1997-05-01), pages 139-149, XP002034740 ISSN: 0167-4412

## Description

### FIELD OF THE INVENTION

The invention refers to the production of peptides and proteins of interest in host system plants by accumulation thereof in endoplasmic reticulum-derived protein bodies, to nucleic acid encoding such products and to the use of said nucleic acids in the manufacture of constructs and vectors for transforming host plant systems. A method for expressing and isolating heterologous products of interest, such as calcitonin (CT), in plants, is specifically disclosed.

### BACKGROUND OF THE INVENTION

As the demand for biopharmaceuticals is expected to increase considerably because of the remarkable advances in genome knowledge and in related biomedical research, there is a considerable interest in elaborating low cost recombinant production systems.

Genetic engineering of plants to produce biopharmaceuticals is relatively recent since other transgenic systems including bacteria, fungi and cultured mammalian cells, have been largely and for a long time adopted for bioproduction. Nevertheless, some recombinant therapeutic proteins using plant expression system are already on the market or in various stages of human clinical trials like hirudin, an anticoagulant protein to treat thrombosis (Parmenter et al, 1995), a chimeric IgG-IgA vaccine against dental caries (Ma et al, 1998), a bacterial vaccinogen against an enterotoxigenic strain of *E. coli* (Haq et al., 1995), and a recombinant dog gastric lipase to treat cystic fibrosis (Bénicourt et al., 1993).

Plant expression systems are attractive because expression level of recombinant proteins can be enhanced by exploiting the innate sorting and targeting mechanisms that plants use to target host proteins to organells. Moreover plant-derived biopharmaceuticals can be easily scale up for mass production and have the advantage to minimize health risks arising from contamination with pathogens or toxins.

Plants, more and more, appear to be an attractive expression system because of their potential to provide unlimited quantities of biologically active material at low production costs and with reduced health risks. The capacity of plants to accumulate high levels of recombinant proteins and to perform most of post translational modifications make them considered as bioreactors for molecular farming of recombinant therapeutics (for review see Fischer and Emans, 2000). However, important decisions concerning crop species selection, tissues choice, expression and recovery strategies and posttranslational processing are determinant for the feasibility of plant-based production toward commercialization (Cramer et al., 1999).

Subcellular targeting of recombinant proteins is an important consideration for high level accumulation and correct assembly and folding of such proteins in plants. Compartimentalization of host proteins into intracellular storage organelles is generally achieved using appropriate signal peptides or whole protein fusions. A variety of recombinant therapeutic proteins have been adressed to the following compartiments of plants, apoplastic space (McCormick et al., 1999), chloroplasts (Staub et al., 2000), endoplasmic reticulum (ER) (Stoger et al., 2000). Immunoglobulins directed to the ER compartment in transgenic plants have been showed to give 10-100 fold higher yields than when adressed to others compartments such as the apoplasm or the cytosol (Conrad and Fiedler, 1998).

The targeting of complex proteins such as antibodies in the ER compartiment is particularly interesting because most of the post-translational modifications required to obtain a functional product take place inside the ER (Düring et al., 1990; Ma and Hein, 1995; Conrad and Fiedler, 1998). Indeed, within the ER, the signal peptide is cleaved and stress proteins such as the binding IgG protein (BiP) and enzymes such as protein disulphide isomerase (PDI), function as chaperones, bind to the unassembled protein and direct subsequent folding and assembly. In addition to these particular characteristics, available evidence indicates that plant ER is highly flexible making it an ideal reservoir for heterologous pharmaceutical proteins. The ER, even if it appears to be the gateway to the secretory pathway, is also able to store proteins for short or long periods of time. Plants store amino acids for long periods in form of specific storage proteins. One mechanism to protect these storage proteins against uncontrolled premature degradation is to deposit them into ER-derived storage organelles called protein bodies (PB) (for review, Müntz, 1998). The assembly of such organelles as the simple accumulation of recombinant proteins into the ER lumen requires as a first step the retention of the host protein. Secretory proteins when correctly folded and assembled into the ER have a variety of cellular destinations mostly by progression via the Golgi apparatus. However, ER retention of soluble transport-competent proteins can be induced by the carboxy-terminal retention/retrieval signal KDEL (or HDEL) (Munro et al., 1987; Wandelt et al., 1992; Vitale et al., 1993). This conserved C-terminal motif, recognized in the Golgi apparatus through transmembrane receptors, permit the recycling of escaped ER resident proteins back to the ER (Vitale and Denecke, 1999; Yamamoto et al., 2001). Many recombinant antibody fragments have been extended with the KDEL signal in order to be stably accumulated in plants ER (Verch et al., 1998; Torres et al., 1999). An alternate way to generate retention and accumulation of recombinant proteins into the ER compartment is to create an appropriate fusion with a natural ER resident such as a seed storage protein.

WO 01/75312 discloses a method for producing a cytokine in a plant host system wherein said plant host system has been transformed with a chimeric nucleic acid sequence encoding said cytokine, said chimeric nucleic acid sequence comprising a first nucleic acid sequence capable of regulating the transcription in said plant host system of a second nucleic acid sequence wherein said second nucleic acid sequence encodes a signal sequence that is linked in reading frame to a third nucleic acid sequence encoding a cytokine and a fourth nucleic acid sequence linked in reading frame to the 3' end of said third nucleic acid sequence encoding a "KDEL" amino acid sequence.

Zeins are a group of proteins that are synthesized during endosperm development in corn and may be separated in four groups α, β, γ and δ, based on their solubility. Zeins can aggregate into PB directly in the ER. Plants or plant tissues comprising rumin stable protein bodies expressed as fusion proteins comprising a full-length zein protein and an operably linked proteinaceous material have been disclosed (WO 00/40738).

γ-Zein, a maize storage protein, is one of the four maize prolamins and represents 10-15% of the total protein in the maize endosperm. As other cereal prolamins, α and γ-zeins are biosynthesized in membrane-bound polysomes at the cytoplasmic side of the rough ER, assembled within the lumen and then sequestrated into ER-derived PB (Herman and Larkins, 1999, Ludevid et al., 1984, Torrent et al., 1986). γ-Zein is composed of four characteristic domains i) a peptide signal of 19 amino acids, ii) the repeat domain containing eight units of the hexapeptide PPPVHL (53 aa), iii) the proX domain where prolines residues alternate with others amino acids (29 aa) and iv) the hydrophobic cysteine rich C-terminal domain (111aa). The ability of γ-zein to assemble in ER-derived PBs is not restricted to seeds. In fact, when γ-zein gene was constitutively expressed in transgenic Arabidopsis plants, the storage protein accumulated within ER-derived PBs in leaf mesophyl cells (Geli et al, 1994). Looking for a signal responsable for the γ-zein deposition into the ER-derived PB (prolamins do not have KDEL signal), it has been demonstrated that the proline-rich N-terminal domain including the tandem repeat domain was necessary for ER retention and that the C-terminal domain was involved in PB formation. However, the mechanisms by which these domains promote the PB assembly are still unknown.

Calcitonin (CT), a 32 amino acids hormonal peptide is essential for correct calcium metabolism and has found widespread clinical use in the treatment of osteoporosis, hypercalcemic shock and Paget's disease (Reginster et al., 1993; Azria et al., 1995; Silverman et al., 1997). Human CT is synthetized as a preproprotein with a signal peptide of 25 amino acids and two propeptides at the N- and C-terminus (57 aa and 21 aa respectively). The resultant active peptide is 32 amino acids long with a single disulphide bridge (Cys1-Cys7) and is amidated at the carboxy terminus. *In vitro,* human CT aggregates which limits its usefulness as a therapeutic. Consequently, salmon CT which is less prone to aggregate is commonly used instead (Cudd et al., 1995). Production of CT is currently achieved by chemical synthesis but the cost of this production encouraged some research groups to explore alternative approaches. Human and salmon CT have been produced in *E. coli* (Ray et al., 1993; Hong et al., 2000), in mouse pituitary cells (Merli et al., 1996), in nonendocrine cell lines Cos-7 and CHO (Takahashi et al., 1997) and more recently in the milk of transgenic rabbits (McKee et al., 1998). Production of bioactive calcitonin by biotechnological methods requires at least two processing steps: i) generation of a glycine-extended calcitonin (Bradbury et al., 1988) and ii) formation of a carboxy-terminal prolinamide *via* the action of the amidation enzyme, peptidyl glycine α-amidating monooxygenase (PAM) (Eipper et al., 1992). Since it is not currently known whether the carboxyl-amidation occurs in plant cells, *in vitro* amidation of plant glycine-extended calcitonin with the PAM enzyme would provide the C-terminal amide (Ray et al., 1993).

### SUMMARY OF THE INVENTION

The problem to be solved by the present invention is to provide an alternate system for producing peptides and proteins of interest in a plant host system.

The solution presented herein is based on the ability of proline rich domains of γ-zein to self-assemble and to confer stability to fusion proteins in the ER of a host plant system. The use of a γ-zein fusion protein based-system to accumulate a product of interest in a host system plant constitutes a successful approach to accumulate said product of interest within ER-derived PBs of plants.

The invention is illustrated in the Example, wherein a fusion protein based-system to accumulate recombinant CT in ER-derived PBs in tobacco plants is described. Various proline rich domains were engineered from γ-zein to serve as fusion partners through a cleavable protease site. Mature calcitonin coding region was fused at the C-terminus of the γ-zein domains and expressed in transgenic tobacco plants. The fusion proteins were accumulated in ER-derived PBs in tobacco leaves. After purification, the fusion proteins were submitted to enterokinase cleavage permitting the release of calcitonin.

Accordingly, an aspect of this invention relates to a nucleic acid sequence comprising (i) a nucleotide sequence encoding the protein γ-zein, or a fragment thereof containing a nucleotide sequence that encodes an amino acid sequence capable of directing and retaining a protein towards the endoplasmic reticulum; (ii) a nucleotide sequence encoding an amino acid sequence that is specifically cleavable by enzymatic or chemical means; and (iii) a nucleotide sequence encoding a product of interest; wherein said nucleotide sequences are operatively linked among them.

In another aspect, this invention relates to a nucleic acid construct comprising said nucleic acid sequence.

In a further aspect, the invention relates to a vector containing said sequence or construct and to a cell transformed with said vector.

In a further aspect, the invention relates to a transformed plant host system having said nucleic acid sequence, construct or vector.

In a further aspect, the invention relates to a transgenic plant host system comprising, integrated in its genome, said nucleic acid sequence.

In a further aspect, the invention relates to a method for producing a product of interest in a plant host system.

In a further aspect, the invention relates to a method for producing calcitonin in a plant host system.

In a further aspect, the invention relates to a fusion protein, said fusion protein having an amino acid sequence corresponding to the above mentioned nucleic acid sequence.

### BRIEF DESCRIPTION OF DRAWINGS

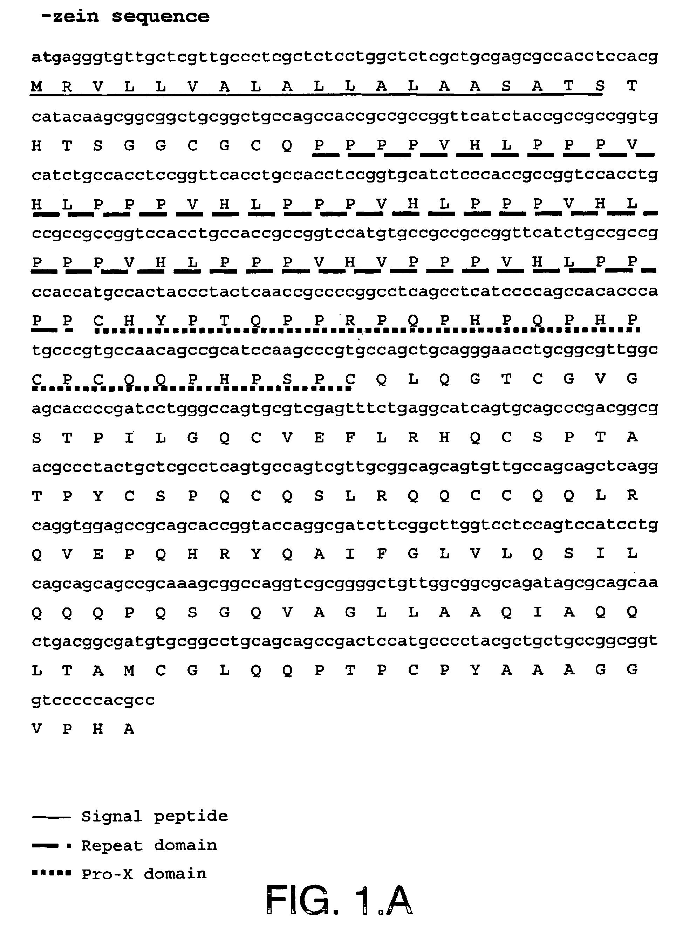

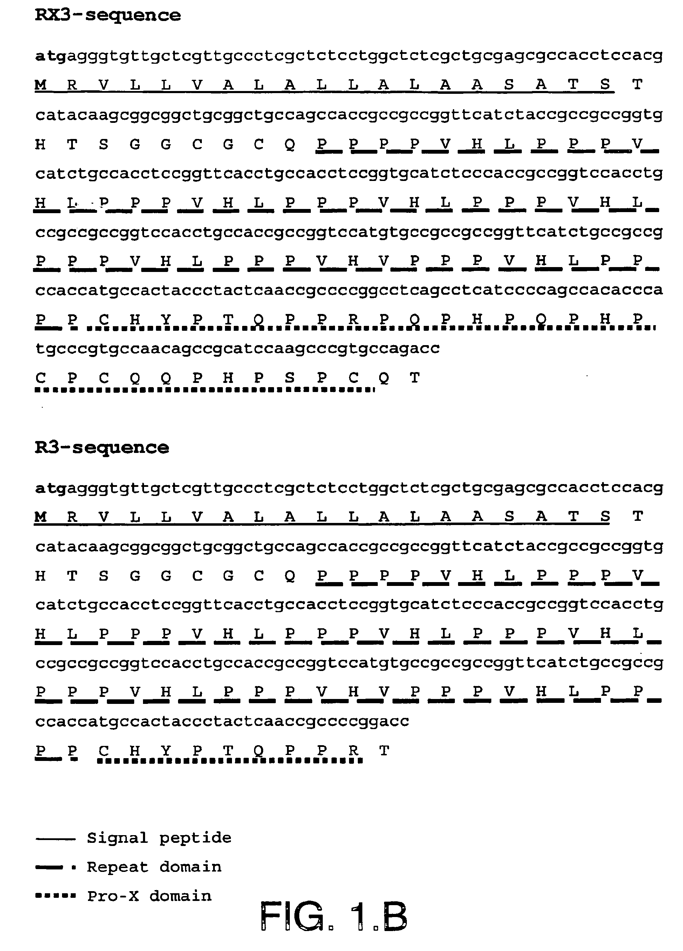

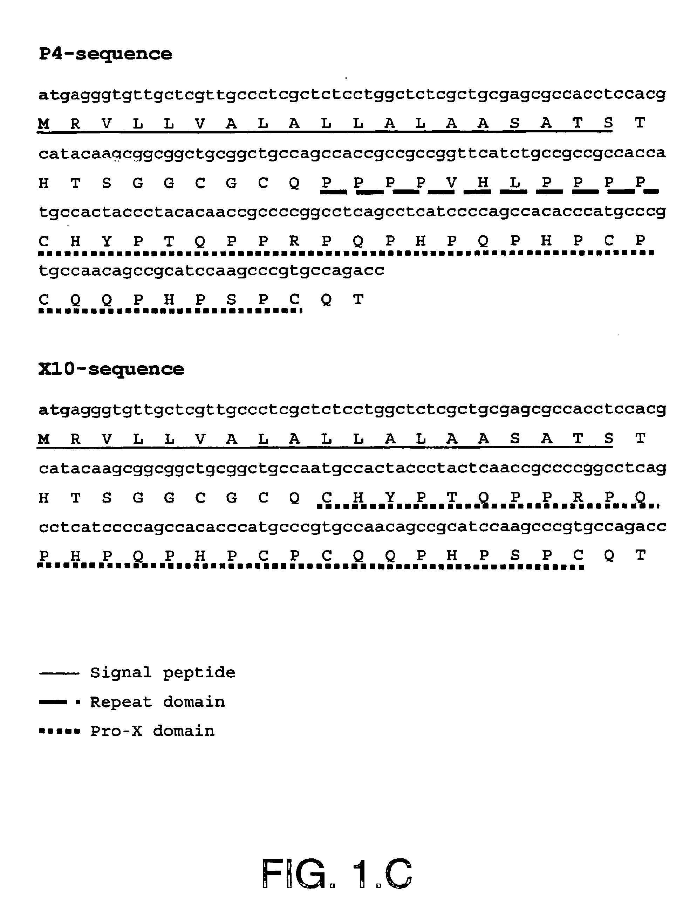

Figure 1 shows the nucleotide sequences and translations of γ-zein (Figure 1.A) and γ-zein derivatives RX3 (Figure 1.B, upper), R3 (Figure 1.B, lower), P4 (Figure 1.C, upper) and X10 (Figure 1.C, lower).

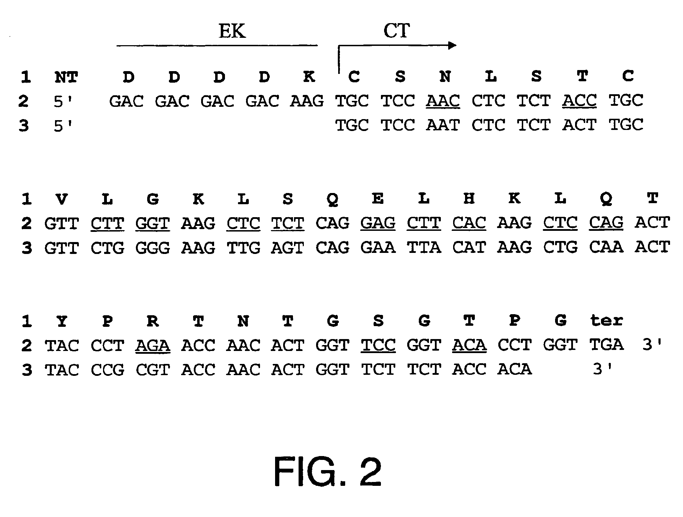

Figure 2 shows the nucleotide sequence (lane 2) and translation (lane 1) of synthetic calcitonin (CT). The synthethic CT gene was constructed using preferential plant codon usage. Codon modifications are underlined in comparation to the wild type salmon CT gene (lane 3). The synthetic gene contains at 5' end a linker sequence corresponding to the enterokinase cleavage site (EK) and is extended at 3' to produce a single C-terminal glycine.

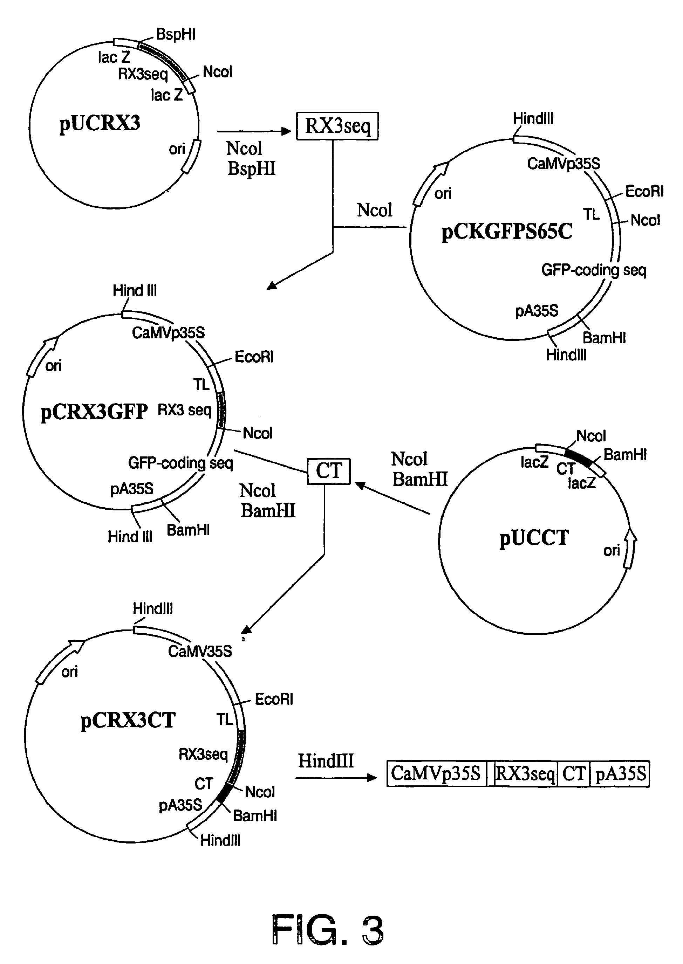

Figure 3 shows a schematic outline for the construction of pCRX3CT plasmid. The process represented was the same for the obtention of the following plasmids pCZeinCT, pCR3CT pCP4CT and pCX10CT, the difference among them being the corresponding γ-zein or γ-zein derived sequences introduced. The different plasmids are not depicted in proportion.

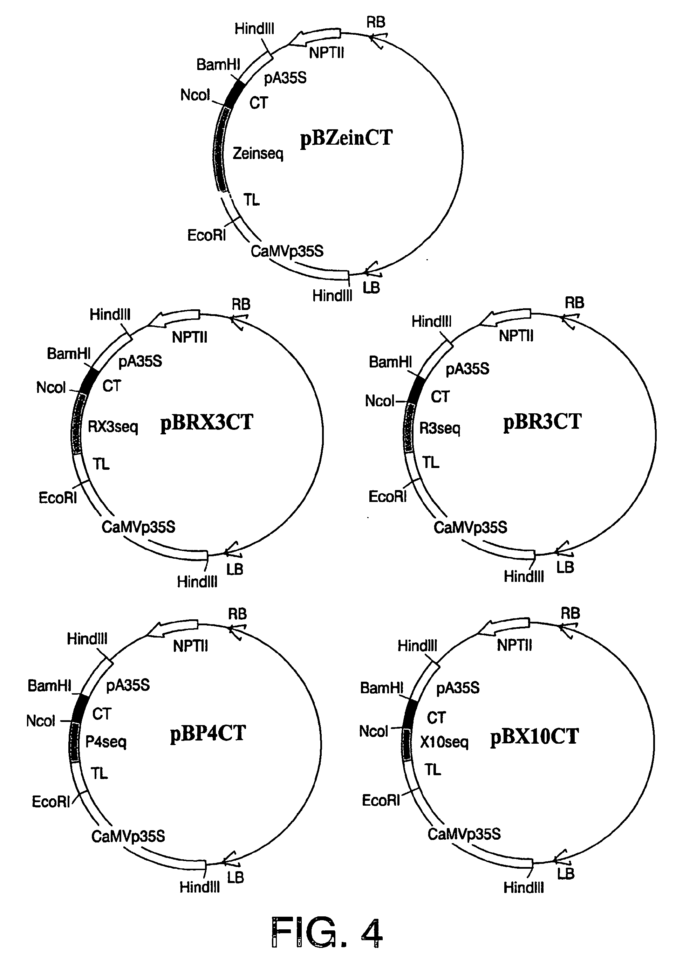

Figure 4 shows a schematic representation of plasmids pBZeinCT, pBRX3CT, pBR3CT, pBP4CT and pBX10CT. The different plasmids are not depicted in proportion.

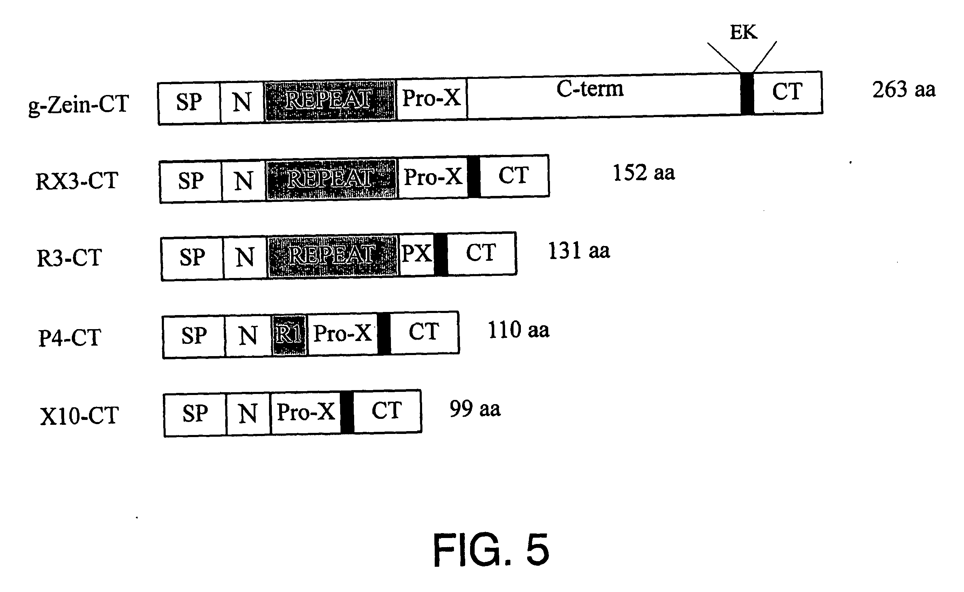

Figure 5 shows a schematic representation of the different fusions proteins. γ-Zein and γ-zein derived domains (RX3, R3, P4 and X10) were fused to calcitonin (CT) through the enterokinase cleavable site (EK). SP, signal peptide; REPEAT, repeat domain (PPPVHL) eight units; R1, one repeat unit; Pro-X, proline-Xaa; PX, fragment of Pro-X domain; C-term, cysteine rich C-terminal domain; N, N-terminal sequence of the mature protein. Amino acids number for each fusion protein is indicated at rigth.

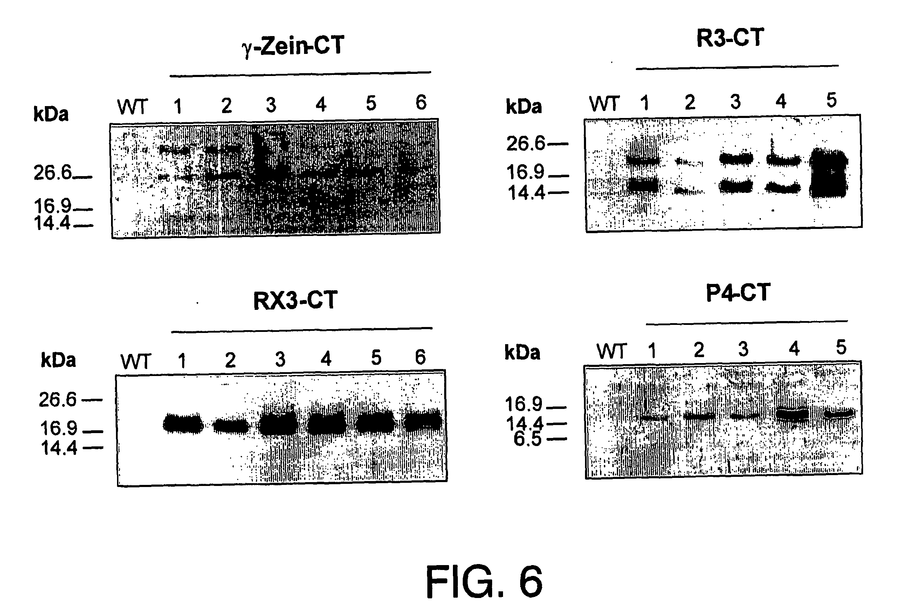

Figure 6 shows the results of an immunoblot analysis of the fusion proteins in transgenic tobacco plants using γ-zein antiserum. Soluble proteins were extracted from wild type (WT) and transgenic tobacco (To) leaves, separated on 15% SDS-polyacrylamide gels (20 µg per lane) and transferred to nitrocellulose. Numbers represent the independant transgenic lines obtained for the different chimeric genes, γ-zein-CT, RX3-CT, R3-CT, P4-CT.

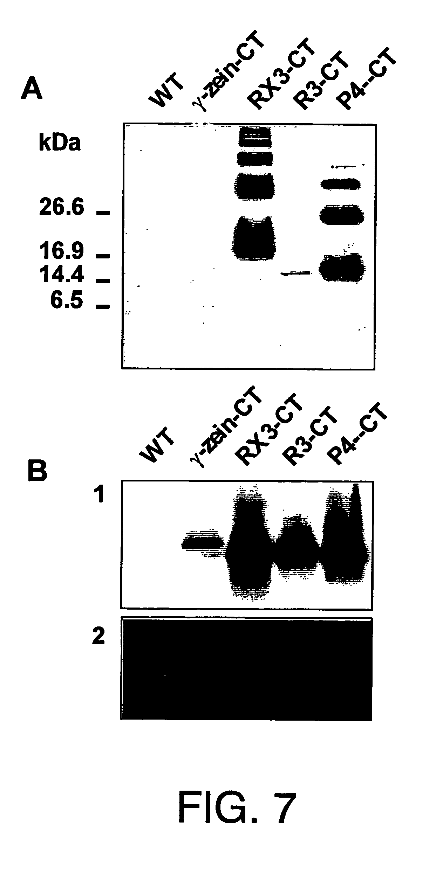

Figure 7 shows: A. Comparative western blot analysis of the different recombinant fusion proteins using CT antiserum. Soluble protein extracts were prepared from wild type plants (WT) and transgenic tobacco lines (T1) having the maximum fusion protein expression of the related chimeric gene. 8 µg of soluble proteins were loaded on 15% SDS-polyacrylamide gel and transferred to nitrocellose. B. Comparative northern blot analysis of the different chimeric gene transcripts. Total RNAs were isolated from the transgenic lines analyzed by immunoblot (Figure 7A), fractionated on denaturing formamide gel electrophoresis (30 µg per lane) and capillary blotted onto nylon membrane. Blots were hybridized with a random primed probe (129 bases) obtained from calcitonin cDNA.

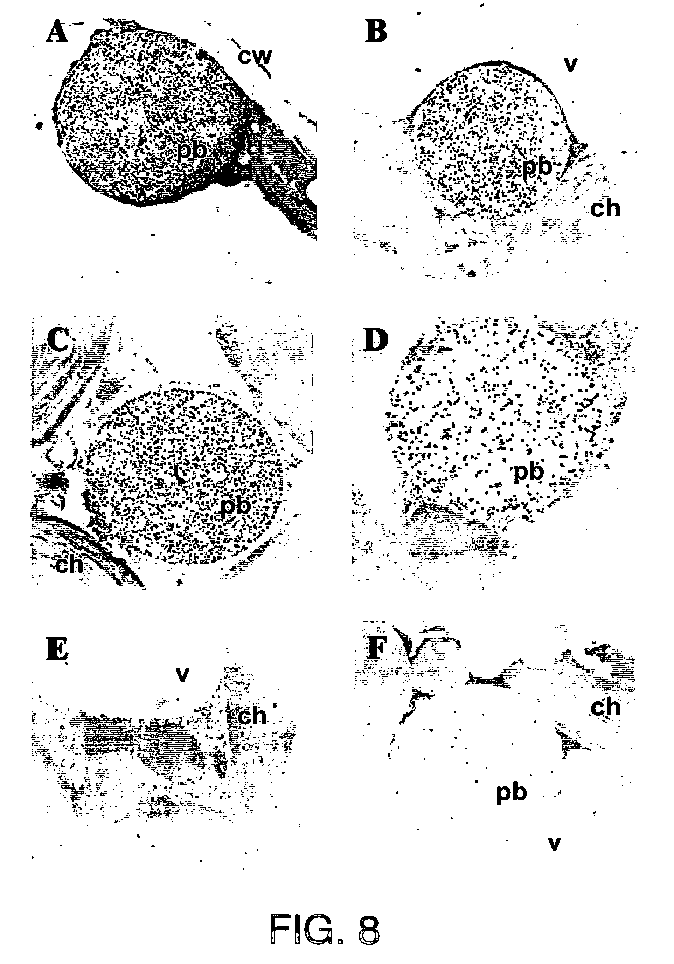

Figure 8 shows the subcellular localization of RX3-CT and P4-CT proteins in transgenic tobaco plants: (A) Immunolocalization of RX3-CT protein in RX3-CT transgenic lines using CT antiserum (dilution 1:100). (B) Immunolocalization of P4-CT protein in P4-CT transgenic lines using CT antiserum (dilution 1:100). (C) Immunolocalization of RX3-CT protein in RX3-CT transgenic lines using γ-zein antiserum (dilution 1:1.500). (D) Immunolocalization of BiP protein in RX3-CT transgenic lines using BiP antiserum (dilution 1:250). (E) Immunolocalization in wild type plants using γ-zein antiserum (dilution 1:1.500). serum. (F) Immunolocalization in RX3-CT transgenic plants without primary antibody (dilution 1:1.500). Immunocytochemistry on tobacco leaf sections was performed by using the primary antibodies indicated and protein A-colloidal gold (15 nm).cw: cell wall; ch: chloroplast; pb: protein body; v: vacuole.

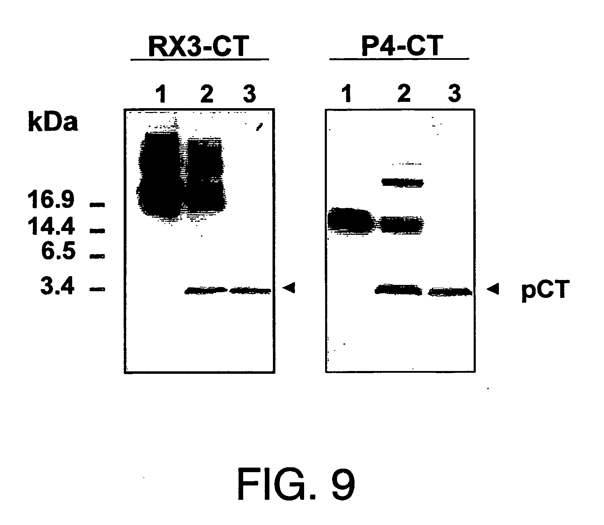

Figure 9 shows the results of the immunoblot analysis of RX3-CT and P4-CT fusion protein EK cleavage. 12 µg of each partially purified fusion protein were incubated with 0.2 U EK during 24 hours at 20°C. Digested fusion proteins were fractionated on 18% Tris-Tricine polyacrylamide gel electrophoresis and transferred to nitrocellulose. Lanes 1, non digested fusion proteins (1 µg); lanes 2, digestion products; lanes 3, synthetic salmon CT standard.

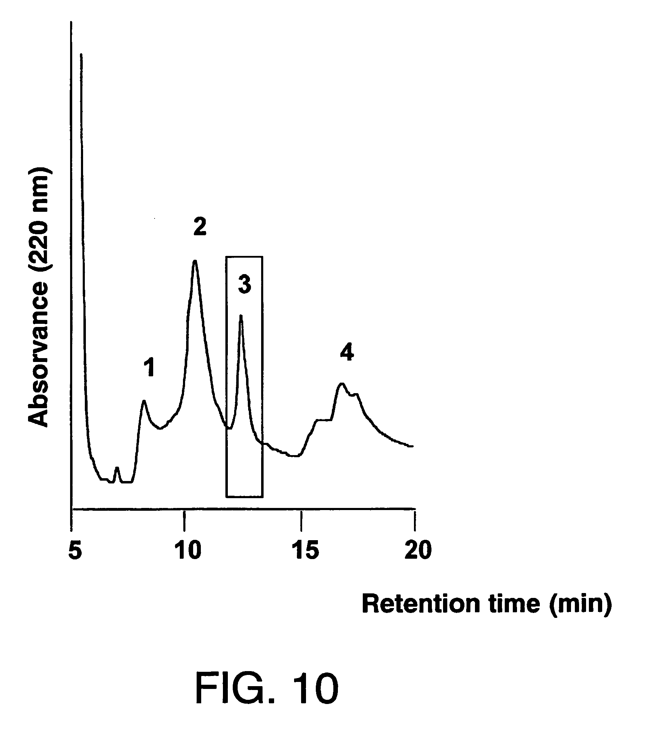

Figure 10 shows the results of RP-HPLC fractionation of RX3-CT fusion protein digested by EK. pCT released from RX3-CT fusion protein was detected in fraction 3 (Tr = 13 min) by TOF-MALDI using synthetic salmon CT as standard.

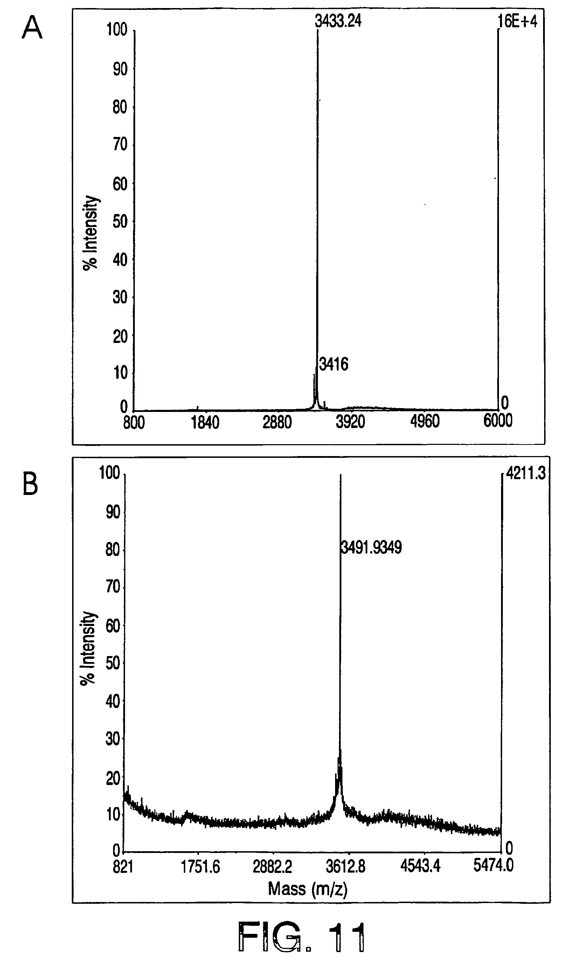

Figure 11 shows the results of TOF-MALDI mass spectrometry characterization of (A) synthetic salmon CT (MW = 3433.24) and (B) plant CT (MW = 3491.93) eluted at Tr = 13 min from the RP-HPLC fractionation.

### DETAILED DESCRIPTION OF THE INVENTION

The first aspect of the invention provides a nucleic acid sequence, hereinafter referred to as the nucleic acid sequence of the invention, comprising:
a first nucleic acid sequence containing the nucleotide sequence that encodes the protein γ-zein, or a fragment thereof containing a nucleotide sequence that encodes an amino acid sequence capable of directing and retaining a protein towards the endoplasmic reticulum (ER);
a second nucleic acid sequence containing a nucleotide sequence that encodes an amino acid sequence that is specifically cleavable by enzymatic or chemical means; and
a third nucleic acid sequence containing the nucleotide sequence that encodes a product of interest;
wherein the 3' end of said first nucleic acid sequence is linked to the 5' end of said second nucleic acid sequence and the 3' end of said second nucleic acid sequence is linked to the 5' end of said third nucleic acid sequence.

The first nucleic acid sequence contains the nucleotide sequence that encodes the protein γ-zein, or a fragment thereof containing a nucleotide sequence that encodes an amino acid sequence capable of directing and retaining a protein towards the ER.

The term "γ-zein" as used herein refers to a maize storage protein which is composed of the four characteristic domains mentioned previously in the Background of the Invention. Said term includes native γ-zein proteins, as well as variants thereof and recombinant γ-zein proteins which are capable of directing and retaining a protein towards the ER.

Practically any nucleotide sequence encoding a γ-zein protein, or a fragment thereof containing a nucleotide sequence that encodes an amino acid sequence capable of directing and retaining a protein towards the ER, may be used.

Accordingly, in a preferred embodiment, the first nucleic acid sequence contains the nucleotide sequence encoding the full-length γ-zein protein. In a particular embodiment, the nucleotide sequence encoding a full-length γ-zein protein is shown in Figure 1A and identified in SEQ ID NO: 1.

In another preferred embodiment, the first nucleic acid sequence contains a nucleotide sequence encoding a fragment of γ-zein protein, said fragment containing a nucleotide sequence that encodes an amino acid sequence capable of directing and retaining a protein towards the ER. In this case, the first nucleic acid sequence may contain:
- one or more nucleotide sequences encoding all or part of the repetition domain of the protein γ-zein;
- one or more nucleotide sequences encoding all or part of the ProX domain of the protein γ-zein; or
- one or more nucleotide sequence encoding all or part of the repetition domain of the protein γ-zein, and one or more nucleotide sequence encoding all or part of the ProX domain of the protein γ-zein.

In a particular embodiment, said first nucleic acid sequence contains a nucleotide sequence encoding a fragment of γ-zein protein, said fragment containing a nucleotide sequence that encodes an amino acid sequence capable of directing and retaining a protein towards the ER, selected from the group consisting of:
- the nucleotide sequence shown in SEQ ID NO: 2 [nucleotide sequence identified as RX3 (Figure 1B)],
- the nucleotide sequence shown in SEQ ID NO: 3 [nucleotide sequence identified as R3 (Figure 1B)],
- the nucleotide sequence shown in SEQ ID NO: 4 [nucleotide sequence identified as P4 (Figure 1C)], and
- the nucleotide sequence shown in SEQ ID NO: 5 [nucleotide sequence identified as X10 (Figure 1C)].

The second nucleic acid sequence contains a nucleotide sequence that encodes an amino acid sequence that is specifically cleavable by enzymatic or chemical means. In a particular embodiment, said second nucleic acid sequence comprises a nucleotide sequence that encodes a protease cleavage site, for example, an amino acid cleavable site by a protease such as an enterokinase, Arg-C endoprotease, Glu-C endoprotease, Lys-C endoprotease, factor Xa and the like.

Alternatively, the second nucleic acid sequence comprises a nucleotide sequence that encodes an amino acid that is specifically cleavable by a chemical reagent, such as, for example, cyanogen bromide which cleaves methionine residues, or any other suitable chemical reagent.

The second nucleic acid sequence may be generated as a result of the union between said first nucleic acid sequence and said third nucleic acid sequence. In that case, each sequence contains a number ofnucleotides in such a way that when said first and third nucleic acid sequences become linked a functional nucleotide sequence that encodes an amino acid sequence that is specifically cleavable by enzymatic or chemical means, i.e., the second nucleic acid sequence, is formed. In an alternate embodiment, the second nucleic acid sequence is a foreign sequence operatively inserted between said first and third nucleic acid sequence.

The third nucleic acid sequence contains the nucleotide sequence that encodes a product of interest. In principle, any product of interest may be expressed by the system provided by the instant invention. In a preferred embodiment, the product of interest is a proteinaceous (i.e., a protein or peptide) drug, for example, a peptide hormone, such as calcitonin, erythropoietin, thrombopoietin, growth hormone and the like, an interferon, i.e., a protein produced in response to viral infections and as cytokine during an immune response, etc. Preferably, said therapeutic products of interest are effective for treating the human or animal body.

In a particular embodiment, the third nucleic acid sequence comprises a nucleotide sequence encoding calcitonin (CT), for example, human calcitonin (hCT) or salmon calcitonin (sCT). In general, in this case, said third nucleic acid sequence, preferably, includes a codon for glycine at the 3' end of said nucleic acid sequence encoding calcitonin thus rendering a glycine-extended calcitonin.

According to the invention, the 3' end of said first nucleic acid sequence is linked to the 5' end of said second nucleic acid sequence and the 3' end of said second nucleic acid sequence is linked to the 5' end of said third nucleic acid sequence, i.e., said first, second and third nucleic acid sequences are in reading frame.

The nucleic acid sequence of the invention may be obtained by using conventional techniques known for the skilled person in the art. In general, said techniques involve linking different fragments of the nucleic acid sequence of the invention in a suitable vector. A review of said conventional techniques may be found, for example, in "Molecular cloning, a Laboratory Manual", 2nd ed., by Sambrook et al., Cold Spring Harbor Laboratory Press, 1989. The construction of some vectors containing a nucleic acid of the invention is disclosed in the Example and illustrated in Figures 3 and 4. As shown therein, various proline rich domains were engineered from γ-zein to serve as fusion partners through a cleavable protease site. Mature calcitonin coding region (32 aa) was fused at the C-terminus of the γ-zein domains and expressed in transgenic tobacco plants. The fusion proteins were accumulated in ER-derived protein bodies in tobacco leaves. After purification, the fusion proteins were submitted to enterokinase cleavage permitting the release of calcitonin which may be further purified from digestion mixture by a reverse phase chromatography.

In another aspect, the invention provides a fusion protein, hereinafter referred to as the fusion protein of the invention, comprising (i) the amino acid sequence of the protein γ-zein, or a fragment thereof capable of directing and retaining a protein towards the ER, (ii) an amino acid sequence that is specifically cleavable by enzymatic or chemical means, and (iii) a product of interest; said fusion protein being the expression product of the nucleic acid sequence of the invention in a host plant system.

The fusion protein of the invention is accumulated in stable, ER-derived PBs, in a host plant system. The enzymatically or chemically cleavable site, which is present at the C-terminus of γ-zein domains, allows to recover the product of interest afterwards. The product of interest may be then isolated and purified by conventional means. Therefore, the fusion protein of the invention constitutes a novel and successful approach to accumulate a product of interest.

In an embodiment, the fusion protein of the invention comprises a full-length γ-zein protein. A specific amino acid sequence of full-length γ-zein is shown in Figure 1A and identified in SEQ ID NO: 6.

In another embodiment, the fusion protein of the invention comprises a fragment of a γ-zein protein, said fragment containing an amino acid sequence capable of directing and retaining a protein towards the ER. In a particular embodiment, the fusion protein of the invention comprises a fragment of a γ-zein protein, selected from the group consisting of:
- the amino acid sequence shown in SEQ ID NO: 7 [amino acid sequence corresponding to RX3 (Figure 1B)],
- the amino acid sequence shown in SEQ ID NO: 8 [amino acid sequence corresponding to R3 (Figure 1B)],
- the amino acid sequence shown in SEQ ID NO: 9 [amino acid sequence corresponding to P4 (Figure 1C)], and
- the amino acid sequence shown in SEQ ID NO: 10 [amino acid sequence corresponding to X10 (Figure 1C)].

The fusion protein of the invention contains an amino acid sequence that is specifically cleavable by enzymatic or chemical means. In a particular embodiment, said cleavable site comprises a protease cleavage site, for example, an amino acid cleavable site by a protease such as an enterokinase, Arg-C endoprotease, Glu-C endoprotease, Lys-C endoprotease, factor Xa and the like, or an amino acid cleavable site by a chemical reagent, such as, for example, cyanogen bromide which cleaves methionine residues, or any other suitable chemical reagent.

The fusion protein of the invention also contains a product of interest, for example, a proteinaceous (i.e., a protein or peptide) drug, such as a peptide hormone, an interferon, and the like. Preferably, said product of interest is effective for treating the human or animal body. In a particular embodiment, the fusion protein of the invention comprises a calcitonin (CT), for example, an optionally glycine-extended human calcitonin (hCT) or salmon calcitonin (sCT).

In a further aspect, the invention provides a nucleic acid construct comprising (i) the nucleic acid sequence of the invention, and (ii) a regulatory nucleotide sequence that regulates the transcription of the nucleic acid of the invention (i), said regulatory sequence (ii) being functional in plants. Said nucleic acid sequences are operatively linked.

Practically any plant functional regulatory sequence may be used. In an embodiment, said regulatory sequence (ii) is, preferably, tissue-specific, i.e., it can regulate the transcription of the nucleic acid of the invention in a specific tissue, such as seeds, leaves, tubercles, etc.

The regulatory sequence (ii) may comprise a promoter functional in plants. Virtually, any promoter functional in plant may be used. In a particular embodiment, said regulatory sequence (ii) comprises the promoter 35SCaMV. In other particular embodiment, said regulatory sequence (ii) comprises the "patatina" promoter, a storage protein promoter, the ubiquitine gene promoter, the regulatory sequences of the γ-zein gene, or the like.

The regulatory sequence (ii) may also contain a transcription termination sequence. Virtually, any transcription termination sequence functional in plant may be used. In a particular embodiment, said transcription termination sequence comprises the terminator 35SCaMV, the terminator of the octopine synthase (ocs) gene, the terminator of the nopaline synthase (nos) gene, the terminator of the γ-zein gene, and the like.

The regulatory sequence (ii) may also contain a translation enhancer functional in plant. Virtually, any translation enhancer functional in plant may be used, for example, the promoting sequence for transcription of the tomato etch virus, and the like.

The nucleic acid sequence of the invention, or the construct provided by this invention, can be inserted into an appropriate vector. Therefore, in a further aspect, the invention provides a vector comprising the nucleic acid sequence of the invention or a nucleic acid construct provided by the instant invention. Suitable vectors include plasmids, cosmids and viral vectors. In an embodiment, said vector is suitable for transforming plants. The choice of the vector may depend on the host cell wherein it is to be subsequently introduced. By way of example, the vector wherein the nucleic acid sequence of the invention is introduced may be a plasmid, a cosmid or a viral vector that, when introduced into a host cell, is integrated into the genome of said host cell and is replicated along with the chromosome (or chromosomes) in which it has been integrated. To obtain said vector, conventional methods can be used (Sambrook et al., 1989).

In a further aspect, the invention provides a plant host system, said plant host system having been transformed with the nucleic acid of the invention, or with a construct or a vector provided by the instant invention.

As used herein, the term "plant host system" includes plants, including, but not limited to, monocots, dicots, and, specifically, cereals (e.g., maize, rice, oat, etc.), legumes (e.g., soy, etc.), cruciferous (e.g., *Arabidopsis thaliana,* colza, etc.) or solanaceous (e.g., potato, tomato, tobacco, etc.). Plant host system also encompasses plant cells. Plant cells includes suspension cultures, embryos, merstematic regions, callus tissue, leaves, roots, shoots, gametophytes, sporophytes, pollen, seeds and microspores. Plant host system may be at various stages of maturity and may be grown in liquid or solid culture, or in soil or suitable medium in pots, greenhouses or fields. Expression in plant host systems may be transient or permanent. Plant host system also refers to any clone of such plant, seed, selfed or hybrid progeny, propagule whether generated sexually or asexually, and descendents of any of these, such as cuttings or seeds.
The transformation of plant host systems may be carried out by using conventional methods. A review of the genetic transfer to plants may be seen in the textbook entitled "Ingenieria genética and transferencia génica", by Marta Izquierdo, Ed. Pirámide (1999), in particular, Chapter 9, "Transferencia génica a plantas", pages 283-316.

In a further aspect, the invention provides a transgenic plant host system, engineered to contain a novel, laboratory designed transgene, said transgenic plant host system comprising, integrated in its genome, the nucleic acid of the invention. Said transgenic plant host system may be obtained by means of conventional techniques, for example, through the use of conventional antisense mRNA techniques and/or overexpression (in sense silencing) or others, for example, by using binary vectors or other vectors available for the different plant transformation techniques currently in use. Examples of transgenic plant host systems provided by the present invention include both monocotyledon and dicotyledonous plants, and, specifically, cereals, legumes, cruciferous, solanaceous, etc.

The nucleic acid sequence of the invention is useful for producing a product of interest in a plant host system. Therefore, in a further aspect, the invention provides a method for producing a product of interest in a plant host system, which comprises growing a transformed or transgenic plant host system provided by the instant invention, under conditions that allow the production and expression of said product of interest in the form of a fusion protein. As mentioned above, said fusion protein is accumulated in stable, ER-derived PBs, in said host plant system. The enzymatically or chemically cleavable site, which is present at the C-terminus of γ-zein domains, allows to recover the product of interest afterwards. The product of interest may be then isolated and purified by conventional means. Accordingly, the method provided by the instant invention further comprises, if desired, the isolation and purification of said fusion protein, and, optionally, the release of said product of interest from said fusion protein. The fusion protein is cleaved at the cleavage site by a suitable enzyme or chemical reagent, as appropriate.

In a further aspect, the invention provides a method for producing calcitonin in a plant host system, comprising:
a) transforming a plant host system with an expression vector or with a nucleic acid construct, comprising a regulatory sequence for the transcription of a nucleic acid sequence (nucleic acid sequence of the invention) that consists of:
   a first nucleic acid sequence containing the nucleotide sequence that encodes the protein γ-zein, or a fragment thereof containing a nucleotide sequence that encodes an amino acid sequence capable of directing and retaining a protein towards the endoplasmic reticulum (ER);
   a second nucleic acid sequence containing a nucleotide sequence that encodes an amino acid sequence that is specifically cleavable by enzymatic or chemical means; and
   a third nucleic acid sequence containing the nucleotide sequence that encodes calcitonin;
   wherein the 3' end of said first nucleic acid sequence is linked to the 5' end of said second nucleic acid sequence and the 3' end of said second nucleic acid sequence is linked to the 5' end of said third nucleic acid sequence;
b) generating complete plants from said plant host systems transformed with said expression vector or nucleic acid construct;
c) growing such transformed plants under conditions that allow the production and expression of calcitonin in the form of a fusion protein; and, if desired
d) isolating, purifying said fusion protein and treating said fusion protein in order to release calcitonin.

The invention provides, therefore, a fusion protein based system to accumulate recombinant products of interest in ER-derived PBs in plant host systems. The invention is further illustrated by the following non limitative example.

### EXAMPLE 1

### Production of calcitonin in tobacco plants

A successful example of CT production in tobacco plants is described below. Various proline rich domains were engineered from γ-zein to serve as fusion partners through a cleavable protease site. Mature CT coding region (32 aa) was fused at the C-terminus of the γ-zein domains and expressed in transgenic tobacco plants. A cleavable protease site was introduced at the C-terminus of γ-zein domains to recover pure calcitonin afterwards. This approach provides a high accumulation of fusion proteins within the ER and the formation of ER-derived PBs in tobacco plants. Fusions proteins were highly accumulated in ER-derived PBs in tobacco leaves. The expression level of said fusion proteins reached, in some cases, up to 12.44% of total soluble proteins. After only two purification steps, the fusion proteins were submitted to enterokinase cleavage permitting the release of calcitonin. Pure calcitonin was obtained from digestion mixture by a reverse phase chromatography. Calcitonin product accumulated in tobacco plants was validated by mass spectroscopy. Fusion proteins purification, protease digestion and full characterization of the released plant calcitonin (pCT) are also presented.

### I. EXPERIMENTAL PROCEDURE

### Construction of chimeric genes and vectors

The wild type γ-zein gene and four γ-zein derived sequences named RX3, R3, P4 and X10 encoding different γ-zein domains (Figure 1A, 1B and 1C) were fused with a synthetic CT gene containing an enterokinase digestion site (Figure 2) and introduced in plant transformation vectors as described below and in Figure 3.
γ-Zein, RX3 and R3 cDNA sequences were generated by PCR using pKSG2 (Torrent et al., 1994) as template. X10 cDNA was amplified from pDR20, a plasmid produced from pKSG2 after deletion of the sequence corresponding to the repeat domain. The primers used for the different PCRs were:
for γ-zein cDNA sequence:
   **T1:** 5'TCATGAGGGTGTTGCTCGTTGCCCTC3', and
   **T4:** 5'CCATGGCGTGGGGGACACCGCCGGC3',
for RX3 and X10 cDNA sequences:
   **T1** and
   **T2:** 5'CCATGGTCTGGCACGGGCTTGGATGCGG 3', and
for R3 cDNA sequence:
   **T1** and
   **T3:** 5'CCATGGTCCGGGGCGGTTGAGTAGGGTA3'.
The PCR products were subcloned into a pUC 18 vector (SureClone Ligation Kit, Pharmacia) and the resulting plasmids were named pUCZein, pUCRX3, pUCR3 and pUCX10. The vector pUCP4 which contains the γ- zein derived sequence P4 (Figure 1C) was obtained during the screening of pUCRX3 derived clones. γ-zein, RX3, R3, P4 and X10 cDNA fragments, containing cohesive ends of BspHI and NcoI, were inserted into the vector pCKGFPS65C (Reichel et al., 1996) previously digested with NcoI. This vector was selected because it contains the regulatory sequences for expression in plants and the GFP coding sequence that would be used for parallel targeting studies of γ-zein derived proteins in transgenic plants. The vectors generated, pCZeinGFP, pCRX3GFP, pCR3GFP, pCP4GFP and pCX10GFP contained the following regulatory sequences for expression in plant systems: i) the enhanced 35S promoter derived from the cauliflower mosaic virus (CaMVp35S), ii) the translational enhancer from tomato etch virus (TL) and iii) the transcription-termination sequence from CaMV35S (pA35S). The γ-zein derived/CT chimeric constructs were generated by substitution of the GFP coding sequence with the CT synthetic gene as described below (see Figure 3).
The synthetic gene encoding the 32 amino acids of active salmon CT (Figure 2) were generated from two 122 bases complementary oligonucleotides. The oligonucleotides were designed to use preferential plant codons in order to achieved high expression in plants. The 5' phosphorilated oligonucleotides synthesized using an Applied Biosystems 394 DNA synthesizer had the following sequences:
**Call:**
**CaIII:**
After purification on 12% polyacrylamide gel, 60 pmole of each oligonucleotide were used to form the double-strand molecule. Hybridation mixture heated to 95°C for 5 min was maintained at 70°C for 1 hour and let get cold at room temperature. The synthetic cDNA fragment contained NcoI and XbaI cohesive ends at 5' and 3' terminal respectively. The synthetic CT cDNA included a 5' linker sequence corresponding to the enterokinase specific cleavage site ((Asp)₄-Lys) and was extended at 3' end to produce a single glycine for further amidation of the CT peptide. The NcoI/XbaI CT cDNA was subcloned into a pUC 18 vector and was then inserted into the NcoI and BamHI restriction sites of the vectors pCZeinGFP, pCRX3GFP, pCR3GFP, pCP4GFP and pCX10GFP containing the derived γ-zein coding sequences and deleted from the GFP coding sequence. The resulting constructs were named pCZeinCT, pCRX3CT, pCR3CT, pCP4CT and pCX10CT (Figure 3). Effective plant transformation vectors pBZeinCT, pBRX3CT, pBR3CT, pBP4CT and pBX10CT (Figure 4) were ultimately obtained by inserting the different HindIII/HindIII expression cassettes into the binary vector pBin19 (Bevan, 1984).

### Stable tobacco plants transformation

Binary vectors were transferred into LBA 4404 strains of *Agrobacterium tumefaciens.* Tobacco (*Nicotiana tobaccum,* W38) leaf discs were transformed according to the method of Draper et al. (1988). Regenerated plants were selected on medium containing 200 mg/L kanamycin and transferred to a greenhouse. Transgenic tobacco plants having the highest transgene product levels were cultivated for obtention of T1 generation. Developing leaves (approximately 12 cm long) were harvested, immediately frozen with liquid nitrogen and stored at -80°C for further experiments.

### Extraction and western blot analysis of recombinant proteins

Tobacco leaves were ground in liquid nitrogen and homogenized using 4 ml of extraction buffer (50 mM Tris-HCl pH 8, 200 mM dithiothreitol (DTT) and protease inhibitors (10 µM aprotinin, 1 µM pepstatin, 100 µM leupeptine, 100 µM phenylmethylsulphonyl fluoride and 100 µM E64 [(N-(N-(L-3-trans-carboxyoxirane-2-carbonyl)-Lleucyl)-agmantine] per gram of fresh leaf material. The homogenates were stirred for 30 min at 4°C and then centrifuged twice (15000 rpm 30 min, 4°C) to remove insoluble material. Total soluble proteins were quantified using the Bradford protein assay (Bio-Rad). Proteins were separed on 15% SDS polyacrylamide gel and transferred to nitrocellulose membranes (0.22 µM) using a semidry apparatus. Membranes were incubated with γ-zein antiserum (dilution 1/7000) (Ludevid et al., 1985) or an antiserum raised against KLH-calcitonin (CT-antiserum) (dilution 1/1000) and were then incubated with horseradish peroxidase conjugated antibodies (dilution 1/10000). Immunoreactive bands were detected by enhanced chemiluminescence (ECL western blotting system, Amersham). Calcitonin antibodies were raised in rabbits by inoculating synthetic salmon calcitonin coupled to KLH. After four inoculations of the antigen (200 g each), the sera was collected, aliquoted and stored at -80°C. Sera titration were carried out by immuno-dot blots using synthetic calcitonin and ELISA assays using BSA-calcitonin as antigen.

### Northern blot analysis

Total RNA was isolated from wild type and transgenic tobacco (T1) leaves according to Logemann et al., 1987. RNA was fractionated on denaturing formamide-agarose gel electrophoresis (30 µg per lane) and was capillary blotted onto nylon membrane (Hybond N, Amersham Pharmacia Biotech). RNA blots were hybridized with a 129 bases DNA probe obtained from CT cDNA and labeled with (α-³²P) dCTP using a random primed DNA labeling kit (Roche). Hybridization was carried out overnight at 42°C and filters were washed three times for 15 min in 3X SSC and 0.5% SDS (W/V) at 65°C. Blots were detected with a phosphorImager scanner (Fluor-S^{™} MultiImager, BIO-RAD).

### ELISA assays

ELISA assays were conducted for plant calcitonin (pCT) quantification on soluble leaf protein extracts and partially purified γ-zein-CT fusion proteins. Microtiter plates (MaxiSorp, Nalgene Nunc International) were loaded with soluble proteins (100 µl) diluted in phosphate-buffered saline pH 7.5 (PBS) and incubated overnight at 4°C. After washing the wells three times, unspecific binding sites were blocked with 3% bovine serum albumin (BSA) in PBS-T (PBS containing 0.1 % Tween 20), one hour at room temperature. The plates were incubated with CT antiserum (dilution 1/1000) for two hours and after four washes with PBS-T, incubated with peroxidase-conjugated secondary antibodies (dilution 1/8000) (Sigma) for two hours. Primary and secondary antibodies were diluted in PBS-T containing 1% BSA. After washing extensively with PBS-T, the enzymatic reaction was carried out at 37°C with 100 µl of substrate buffer (100 mM sodium acetate pH 6, 0.01 mg/ml TMB (3,3',5,5'-tetramethylbenzidine) and 0.01% hydrogen peroxide). The reaction was stopped after 10 min with 2N sulfuric acid and the optical density was measured at 450 nm using a Multiskan EX spectrophotometer (Labsystems). The antigen concentration in plant extracts was extrapolated from a standard curve obtained by using calcitonin-BSA and CT antiserum (dilution 1/1000).

### Electron microscopy

Leaves from wild-type and transgenic plants were fixed by vacuum infiltration with 1% glutaraldehyde and 2.5% paraformaldehyde in 20 mM phosphate buffer, pH 7.4 for one hour at room temperature. After washing with 20 mM phosphate buffer and 200 mM ammonium chloride successively, samples were dehydrated through ethanol series and embedded in Lowicryl K4M resin. Immunochemistry was performed essentially as described by Moore et al., 1991. Ultrathin sections were incubated with antisera against, KLH-calcitonin (1/500), αBiP (1/500) and γ-zein (1/1500). Protein A-colloidal gold (gold particles of 15 nm) was used for antibody detection. As a control, parallel incubations were carried out on non-transgenic plant samples using identical dilutions of primary antibodies and on transgenic samples without primary antibody. Sections were stained with uranyl acetate and lead citrate and examined with a model 301 electron microscope (Phillips, Eindhoven, The Netherlans).

### Purification and enterokinase cleavage of RX3-CT and P4-CT fusion proteins

Soluble extracts of RX3-CT and P4-CT were obtained from leaves of transgenic tobaco plants (T1) in extraction buffer as described above. Solid (NH₄)₂SO₄ was progressively added at 0°C to RX3-CT and P4-CT soluble extracts to 45% and 60% saturation respectively. The samples were stirred for 30 min at 0°C and were then centrifuged at 15000 rpm for 45 min at 4°C. The precipitated proteins were resuspended in 20 mM Tris-HCl pH 8.6 and desalted on PD 10 column (Sephadex G-25 M, Amersham Pharmacia). Desalted protein extracts were fractionated by Fast Performance Liquid Chromatography (FPLC) using an anion exchange column (HiTrap Q sepharose, Amersham Pharmacia) equilibrated with 20 mM Tris-HCl pH 8.6, 100 mM DTT. Protein elution was carried out with a linear salt gradient from 0 to 200 mM NaCl in 20 mM Tris-HCl pH 8.6, 100 mM DTT. The presence of RX3-CT and P4-CT in eluted fractions was assessed by 15% SDS polyacrylamide gel electrophoresis and immunoblot detection using CT antiserum. Positive fractions were desalted and concentrated with 5 K NMWL centrifugal filters (BIOMAX, Millipore). Quantification of RX3-CT and P4-CT fusion proteins was performed by ELISA.
For EK digestion, 15 µg of partially purified fusion proteins were incubated with 0.2 U EK (EK Max, Invitrogen) in 30 µl of digestion buffer (50 mM Tris-HCl pH8, 1 mM NaCl, 0.1% Tween-20) for 24 hours at 20°C. EK digestion buffer was supplemented with 100 mM DTT. The presence of the reducing agent allows to optimize enterokinase cleavage. Digestion products were analysed on 18% Tris-Tricine polyacrylamide gel electrophoresis and released pCT was detected by immunoblot. Synthetic salmon CT was used as positive control.

### Purification and analysis of released pCT

Plant calcitonin (pCT) released from fusion proteins by EK digestion was purified by RP-HPLC. Digestion mixture was applied to an analytical RP-C18 column (250 x 4 mm, 10 µM particule size, 120 Å pore size) and the column was eluted using a gradient ranging from 25 to 60% acetonitrile with 0.036% TFA in 20 min at a flow rate of 1 ml/min. The fractions collected were concentrated by lyophilization and stored at -20°C for pCT characterization. In a separate experiment, standard salmon CT was eluted under the same chromatographical conditions. TOF-MALDI mass spectrometry was used for pCT characterization. RP-HPLC fraction aliquots were mixed with equal volume of a matrix solution (10 mg/ml α-cyano-4-hydroxycinnamic acid and 0.1 % TFA) and 1 µl of the mixure was deposited on the holder and analyzed with a Voyager-DE-RP mass spectrometer (Applied Biosystems). Standard salmon CT was always used in TOF-MALDI mass spectrometry experiments as a control. C-terminal analysis of the pCT was performed by incubating the purified peptide (20 pmoles/µl) for 60 min at 37°C with carboxypeptidase Y (0.1U/µl) and analysis of the digestion products by TOF-MALDI mass spectrometry.

### II. RESULTS

### Construction of several derived γ-zein-CT chimeric genes

The expression and successful assembly of γ-zein proline rich domains into ER-derived protein bodies in plant leaves (Geli et al., 1994) provide a valuable tool to accumulate therapeutic proteins in the ER of plant tissues. γ-Zein gene was deleted to create various proline-rich truncated proteins used as fusion partner to produce CT in tobacco plants. The chimeric genes comprised the γ-zein domains and a CT synthetic gene linked by a linker corresponding to a protease cleavable site. The synthetic gene encoding the 32 amino acids active salmon calcitonin was generated from two complementary oligonucleotides (122 bases) designed to use preferential plant codons in order to achieve high expression of the recombinant peptide in plants. The synthetic CT cDNA (Figure 2) included at 5' end a linker sequence corresponding to the enterokinase cleavage site ((Asp)₄-Lys) and at 3' end an additional codon to produce a glycine. This glycine is a necessary substrate for the amidating enzyme (PAM) to generate the C-terminal prolinamide essential for CT biological activity. The calcitonin cDNA was fused to the sequences encoding the γ-zein domains in a C-terminal fusion. For optimal expression of the derived γ-zein-CT chimeric genes in plant systems, the plant transformation vectors contained the following regulatory sequences i) the constitutive enhanced 35S promoter and the 35S terminator from the cauliflower mosaic virus and ii) the translational enhancer from tomato etch virus (TL). The different fusion proteins generated are represented in Figure 5. The γ-zein-CT fusion protein contains the whole γ-zein fused to CT. The RX3-CT, R3-CT, P4-CT and X10-CT fusion proteins contain the derived γ-zein domains linked to CT in the same way as whole γ-zein. These fusion proteins differ essentially in the presence or the abscence of the repeat and proX domains.

### Production of fusion proteins in tobacco plants

All the fusion genes were used for stably tobacco plant transformation via *Agrobacterium tumefaciens.* At least twenty independant kanamycin-resistant plants (To) were regenerated for each fusion gene. The screening of the transgenic plants was performed by western blot analysis of soluble proteins extracts using a γ-zein polyclonal antiserum. Transgenic lines immunoblot patterns representative of each fusion gene are shown in the Figure 6. As observed, recombinant fusion proteins were obtained in all transgenic lines with the exception of the X10-CT fusion gene where no traces of fusion proteins were detected. This small fusion protein (80 amino acids) is probably unstable in tobacco plants. Two immuno-labelled bands were detected in the R3-CT transgenic lines, one with an atypical high apparent molecular mass. This fusion protein was probably subjected to post-traductional modifications such as glycosylation. Indeed, it has been demonstrated that the γ-zein proline rich repeat domain is able to be glycosilated when expressed in Arabidopsis plants (Alvarez et al., 1998). Protein expression level was quite variable between the different lines of a same fusion gene with the exception of the RX3-CT fusion gene which showed a high recombinant protein expression level in all transgenic lines. An additional immunoblot screening was carried out using an antiserum specifically raised against the sCT peptide (Figure 7 A). As observed, the RX3-CT and the P4-CT proteins were strongly recognized by the sCT antiserum indicating that these fusions provide a better accumulation of the CT peptide in tobacco plants. It could be noted that RX3-CT and P4-CT immunoblot patterns displayed several labelled bands, the major band corresponding to the correct apparent molecular mass of the related recombinant protein. One hypothesis could be that the high molecular weight labelled bands were the result of an oligomerization process on γ-zein domains which formed during the accumulation of the fusion proteins in plants tissues. In order to check the expression levels of fusion genes in relation to protein levels, a comparative northern blot analysis (Figure 7 B) was performed using the transgenic lines analysed by immunoblot in Figure 7 A. As shown, RX3-CT and P4-CT transcripts were the more abundant demonstrating a stable accumulation of these transcripts. Surprisingly, R3-CT transcripts were relatively abundant in comparison to the low R3-CT fusion protein level detected by immunoblot. Probably , the post-translational modification avoid the correct self-assembly of the fusion protein and subsequently its stability in the ER.
The maximum expression level of RX3-CT and P4-CT proteins, measured by ELISA on leaf protein extracts from T1 plants, were respectively 12.44% and 10.65% of total soluble proteins whereas γ-zein-CT and R3-CT expression level remained as lower as 0.01 % of total soluble proteins. With regard to these results, RX3-CT and P4-CT transgenic lines were chosen for further experiments conducing to the production of plant calcitonin (pCT).

### Subcellular localization of fusion proteins RX3-CT and P4-CT

Expression of γ-zein and two γ-zein deletion mutans in Arabidopsis plants demonstrated that these proteins located within the ER of mesophyl cells forming ER-derived PBs (Geli et al., 1994). It was not evident, however, that the calcitonin fused to γ-zein derivatives was sorted to similar organelles, the ER-PBs. To examine the subcellular localization in tobacco leaves of the γ-zein fusion proteins containing calcitonin, the inventors used immunoelectron microscopy (Figure 8). Ultra thin sections of transgenic tobacco leaves expressing RX3-CT and P4-CT proteins, were incubated with CT antibody and protein A-gold. A large PB-like organelles strongly labelled were observed in mesophyl cells of tobacco expressing RX3-CT and P4-CT (Figure 8 A and B, respectively). Few vesicles were detected per cell and their size was quite heterogeneous. Since fusion proteins contained calcitonin protein and γ-zein fragments, the ultrathin sections were also incubated with γ-zein antibody. As was expected, the PBs were labeled with γ-zein antibody confirming that the fusion proteins accumulated inside these organelles (Figure 8 C). To demonstrate that the PBs were formed from the ER, the sections were incubated with an antibody against the ER resident protein, BiP (Figure 8 D). The concomitant occurrence of the CT-fusion proteins and BiP in these organelles indicated that RX3-CT and P4-CT accumulated within the ER lumen to form further independent ER-derived vesicles. Since the inventors were not able to detect PB-like organelles in ultrathin sections of non-transgenic plants (Figure 8 E), the control experiments were performed without primary antibody in transgenic plants (Figure 8 F). As expected no specific label was detected in control experiments.

### Purification of fusion proteins and release of pCT

RX3-CT and P4-CT fusion proteins were effectively extracted from transgenic tobacco leaves (T1) using an extraction buffer including a reducing agent such as DTT (200 mM). About 85 µg of RX3-CT and 73 µg of P4-CT were recovered per gram of fresh material. RX3-CT and P4-CT proteins were concentrated respectively by 45% and 60% ammonium sulfate precipitation. The desalted protein extracts were fractionated by FPLC using an anion exchange chromatography and the recovered fusion proteins were quantified by ELISA. RX3-CT protein represented about 80% of total purified proteins whereas P4-CT was only about 50% of total purified proteins. Such difference could be explained by the fact that more proteins precipitate at 60% of ammonium sulfate than at 45% and that consequently the precipitated P4-CT proteins contained much more contaminant proteins. The partially purified fusion proteins RX3-CT and P4-CT were digested by EK and pCT release was controlled by a Tris-Tricine polyacrylamide gel electrophoresis and immunodetection. As shown in Figure 9, a single labelled band corresponding to calcitonin was generated from both RX3-CT and P4-CT protein cleavage. Small amounts of fusion proteins RX3-CT and P4-CT remained undigested probably due to the non accessibility of the enzyme to some cleavage sites.

### Purification and characterization of pCT

Plant calcitonin (pCT) was isolated by fractionation of the EK digestion mixtures on an analytical C18 RP-HPLC column (Figure 10) and analysis of the eluted fractions by TOF-MALDI mass spectrometry using synthetic sCT as standard (MW 3433.24, Figure 11. A). pCT calcitonin was eluted at 13 min (synthetic sCT Tr = 14 min) and gave a single spectrum with a mass of 3491.93 Da by TOF-MALDI mass spectrometry that is consistent with the theoretical molecular mass of the reduced C-terminal glycine extented calcitonin (Figure 11 B). Mass spectrometry analysis of pCT subjected to carboxypeptidase Y digestion confirmed the integrity of the C-terminal glycine that is essential to produce the C-terminal prolinamide.

### III. DISCUSSION

A successful fusion protein-based system to accumulate salmon calcitonin in tobacco plants is presented. Two fusion proteins RX3-Cal and P4-Cal were found to strongly accumulate in ER-derived PBs of tobacco leaves. These fusion proteins contain the CT peptide and the proline rich domains of γ-zein which consist in i) the repeat domain composed of eight units of the hexapeptide PPPVHL (only one unit in P4-Cal fusion protein) and ii) the proX domain where proline residues alternate with other amino acids. The γ-zein proline rich domains are necessary for the correct retention and assembly of γ-zein within Arabidopsis plants ER (Geli et al., 1994). The folding and the stabilization of the γ-zein polypeptide chains in the ER have been attributed to the ability of the repeat and proX domains to self-assemble and to promote the formation of oligomers. The particular conformation adopted by these highly hydrophobic domains would be due to the proline rich sequences which are able to form an amphipathic secondary structure. As a result of its proper conformation, the proline rich domains would induce aggregation mechanisms involving protein-protein interactions and disulphide cross-links conducing to the ER retention and the formation of ER-derived PBs. This example shows that when expressed in a N-terminal fusion manner the γ-zein proline rich domains conserve the whole capacity to self-assemble and to promote the complex events which lead to the retention and the accumulation in the ER-derived PBs. The salmon CT involved in the fusion protein was also found to greatly accumulate in the PBs. The high expression level of CT in the transgenic tobacco plants can be attributed to the ability of the proline rich domains to fold and to stabilize the fusion protein. The deposition of the fusion protein in the PBs certainly contribute to the enrichment of the plant tissues in CT by removing it from the hydrolytic intracellular environment. As small peptides are unstable in biological systems the fusion protein approach has been currently used to produce calcitonin in heterologous systems, for example in *E. coli* (Ray et al., 1993; Yabuta et al., 1995; Hong et al., 2000), in *Staphylococcus carnosus* (Dilsen et al., 2000) and in the milk of transgenic rabbits (Mckee et al., 1998). In this last case, the fusion of CT with human alpha lactalbumin had also the purpose to mask the calcitonin activity to avoid a possible interference with the normal animal development.

Inventors succeeded in rapid production of glycine extended sCT from tobacco plants:
i) RX3-Cal and P4-Cal fusion proteins were efficiently recovered from tobacco tissues because of their high solubility in the presence of reducing agents,
ii) enterokinase release of calcitonin from the fusion proteins was accomplished after one purification step of the fusion protein by an anion exchange chromatography, and
iii) a reverse phase chromatography led to purified CT by removing it from EK digestion mixture.

Mass spectrometry analysis of the released CT confirmed that correct glycine extended CT was produced by the tobacco plants.

### REFERENCES

Álvarez, I., Gelim M.I., Pimentel, E., Ludevid, D., Torrent, M. 1998. Lysine-rich γ-zeins are secreted in transgenic Arabidopsis plants. Planta 205:420-427.
Azria, M., Copp, D.H., Zanelli, J.M. 1995. 25 years of salmon calcitonin: from synthesis to therapeutic use. Calcif. Tissue Int. 57 (6): 405-408.
Bénicourt, C., Blanchard, C., Carrière, F., Verger, R. and Junien, J.L. 1993. Potential use of a recombinant dog gastric lipase as an enzymatic supplement to pancreatic extracts in cystic fibrosis, pp. 291-295 in Clinical Ecology of Cystic Fibrosis. Escobar H, Baquero CF, Suarez L (eds). Elsevier Sciences Publishers BV, Amsterdam. Bevan, M. 1984. Binary Agrobacterium vectors for plant transformation. Nucleic Acids Research 12: 8711-8721. Bradbury, A.F. and Smyth, D. G. 1988. Biosynthesis of peptide neurotransmitters: studies on the formation of peptide amides. Physiol. Bohemoslov. 37:267-274. Conrad, U. and Fiedler, U. 1998. Compartment-specific accumulation of recombinant immunoglobulins in plant cells: an essential tool for antibody production and immunomodulation of physiological functions and pathogen activity. Plant Mol. Biol. 38: 101-109. Cramer, C.L., Boothe, J.G. and Oishi, K.K. 1999. Transgenic plants for therapeutic proteins linking upstream and downstream strategies. Curr. Top. Microbiol. Immunol. 240: 95-118. Cudd, A., Arvinte, T., Das, RE., Chinni, C. and Maclntyre, I. 1995. Enhanced potency of human calcitonin when fibrillation is avoided. J. Pharm. Sci. 84: 717-719. Dilsen, S., Paul, W., Sandgathe, D., Tippe, R, Freudl, J., Kula, M.R., Takors, C., Wandrey, D. and Weuster-Botz, D. 2000. Fed-batch production of recombinant human calcitonin precursor fusion protein using Staphylococcus carnosus as an expression-secretion system. Appl. Microbiol. Biotechnol. 54: 361-369. Draper, J., Scott, R. and Hamil, J. 1988. In: Plant Genetic Transformation and Gene Expression. A Laboratory Manual (Eds. Draper, J., Scott, R., Armitage, P. and Walden, R.), Blackwell Scientific Publications Düring, K., Hippe, S., Kreuzaler, F. and Schell, J. 1990. Synthesis and self-assembly of a functional monoclonal antibody in transgenic Nicotiana tabacum. Plant Mol. Biol. 15: 281-293. Eipper, B.A., Stoffers D.A. and Mains RE. 1992. The biosynthesis of neuropeptides: peptide α-amidation. Annu. Rev. Neurosci. 15: 57-85. Fischer, R. and Emans, N. 2000. Molecular farming of pharmaceutical proteins. Transgenic Research 9: 279-299. Geli, M.I., Torrent, M. and Ludevid, M.D. 1994. Two structural domains mediate two sequential events in γ-zein targeting: protein endoplasmic reticulum retention and protein body formation. Plant Cell 6: 1911-1922.
Haq, T.A., Mason, H.S., Clements, J.D. and Arntzen, C.J. 1995. Oral immunization with a recombinant bacterial antigen produced in transgenic plants. Science 268: 714-715.
Herman, E.M. and Larkins, B.A. 1999. Protein storage bodies. Plant Cell 11: 601-613.
Hong, D., Mingqiang, Z., Min, L, Changqing, C. and Jifang, M. 2000. production of recombinant salmon calcitonin by amidation of precursor peptide using enzymatic transacylation and photolysis in vitro. Biochem. Biophys. Res.Com. 267: 362-367
Kogan, M.J., Dalcol, I., Gorostiza, P., López-Iglesias, C., Pons, M., Sanz, F., Ludevid, M.D., and Giralt, E. 2001. Self-assembly of the amphipathic helix (VHLPPP)8. A mechanism for zein protein body formation. J. Mol. Biol. 00: 1-7.
Logemann, J., Schell, J., Willmitzer, L. 1987. Improved method for the isolation of RNA from plant tissues. Anal. Biochem. 163: 16-20.
Ludevid, M.D., Martínez-Izquierdo, J.A., Armengol, M., Torrent, M., Puigdomènech, P. and Palau, P., J. 1985. Immunological relations between glutelin-2 and low molecular weight zein-2 proteins from maize (Zea mays) endosperm. Plant sci. 41: 41-48.
Ludevid, M.D., Torrent, M., Martínez-Izquierdo, J.A., Puigdomènech, P. and Palau, P. 1984. Subcellular localization of glutelin-2 in maize. Plant Mol. Biol. 3: 277-234.
Ma, J.K., Hikmat, B.Y., Wycoff, K., Vine, N.D., Chargelegue. D., Yum L., Hein, M.B. and Lehner, T. 1998.Characterization of a recombinant plant monoclonal secretory antibody and preventive immunotherapy in humans. Nat. Med. 4: 601-606.
Ma, J.K. and Hein, M.B. 1995. Plant antibodies for immunotherapy. Plant Physiol. 109: 341-346.
McCormick, A.A., Kumagai, M.H., Hanley, K., Turpen, T.H., Hakim, I., Grill, L.K., Tusé, D., Levy, S. and levy, R. 1999. Rapid production of specific vaccines for lymphoma by expression of the tumor-derived single-chain Fv epitopes in tobacco plants. Proc. Natl. Aca. Sci. USA 96 (2) 703-708.
McKee, C., Gibson, A., Dalrymple, M., Emslie, L., Garner, I. and Cottingham, I. 1998. Production of biologically active salmon calcitonin in the milk of transgenic rabbits. Nature Biotechnology 16: 647-651.
Merli, S., De Falco, S., Verdoliva, A., Tortora, M., Villain, M., Silvi, P., Cassani, G. and Fassina, G. 1996. An expression system for the single-step production of recombinant human amidated calcitonin. Protein Express. Purif. 7: 347-354.
Moore, P.J., Swords, K.M.M., Lynch, M.A. and Staehelin, L.A. 1991. Spatial organization of the assembly pathways of glycoproteins and complex polysaccharides in the Golgi apparatus of plants. J. Cell Biol. 112: 589-602.
Munro, S. and and Pelham, H.R.B. 1987. A C-terminal signal prevents secretion of luminal ER proteins. Cell 48: 899-907.
Müntz, K. 1998. Deposition of storage proteins. Plant Mol. Biol. 38: 77-99.
Parmenter, D.L., Boothe, J.G., Van Rooijen, G.J., Yeung, E.C. and Moloney, M.M. 1995. Production of biologically active hirudin in plant seeds using oleosin partitioning. Plant Mol. Biol. 29: 1167-1180.
Ray, M.V., Van Duyne, P., Bertelsen, A.H., Jackson, Matthews, D.E., Sturmer, A.M., Merkler, D.J., Consalvo, A.P., Young, S.D., Gilligan, J.P. and Shields, P.P. 1993. Production of recombinant salmon calcitonin by in vitro amidation of an Escherichia coli produced precursor peptide. Bio/Technology 11: 64-70.
Reginster, J.Y. 1993. Calcitonin for prevention and treatment of osteoporosis. Am. J. Med. 95 (5A):44S-47S.
Reichel, C., Mathur, J., Eckes, P., Langenkemper, K., Koncz, C., Schell, J., Reiss, B., and Maas, C. 1996. Enhanced green fluorescence by the expression of an Aequorea victoria green fluorescent protein mutant in mono- and dicotyledonous plant cells. Proc. Natl. Acad. Sci. USA 93: 5888-5893.
Sambrook, J., Fritsch, E., Maniatis, T., 1989. Molecular Cloning: A laboratory manual. Cols Spring Harbor Laboratory, Cold Spring Harbor, New York.
Silverman, S.L. 1997. Calcitonin. Am. J. Med. Sci. 313 (1): 13-16.
Staub, J.M., Garcia, B., Graves, J., Hajdukiewicz, P.T., Hunter, P., Nehra, N., Paradkar, V., Schlittler, M., Caroll, J.A., Spatola, L., Ward, D., Ye, G. and Russell, D.A. 2000. High-yield production of a human therapeutic protein in tobacco chloroplasts. Nature Biotechnology 18: 333-338.
Stöger, E., Vaquero, C., Torres, E., Sack, M., Nicholson, L., Drossard, J., Williams, S., Keen, D., Perrin, Y., Christou, P. and Fischer, R. 2000. Cereal crops as viable production and storage systems for pharmaceutical scfv antibodies. Plant Mol. Biol. 42: 583-590.
Takahashi, K.I., Liu, Y.C., Hayashi, N., Goto, F., Kato, M., Kawashima, H. and Takeuchi, T. 1997. Production of bioactive salmon calcitonin from the nonendocrine cell lines COS-7 and CHO. Peptides 18: 439-444.
Torrent, M., Poca, E., Campos, N., Ludevid, M.D. and Palau, J. 1986. In maize, glutelin-2 and low molecular weight zeins are synthesized by membrane-bound polyribosomes and translocated into microsomal membranes. Plant Mol. Biol. 7: 393-403.
Torrent, M., Geli, M.I., Ruir-Avila, L., Canals, JM., Puigdomènech, P. and Ludevid, D. 1994. Role of structural domains for maize g-zein retention in Xenopus oocytes. Planta 192: 512-518.
Torres, E., Vaquero, C., Nicholson, L., Sack, M., Stöger, E., Drossard, J., Christou, P., Fischer, R. and Perrin, Y. 1999. Rice cell culture as an alternative production system for functional diagnostic and therapeutic antibodies. Transgenic Research 8 : 441-449.
Verch, T., Yusibov, V. and Koprowski, H. 1998 Expression and assembly of a full-length monoclonal antibody in plants using a plant virus vector. J. Immunol. Methods 220: 69-75.
Vitale, A., Ceriotti, A. and Denecke, J. 1993. The role of the endoplasmic reticulum in protein synthesis, modification and intracellular transport. J. Exp. Bot. 44: 1417-1444.
Vitale, A. and Denecke, J. 1999. The endoplasmic reticulum-gateway of the secretory pathway. Plant Cell 11: 615-628.
Wandelt, C.I., Khan, M.R.I., Craig, S., Schroeder, H.E., Spencer, D. and Higgins, T.J.V. 1992. Vicilin with carboxy-terminal KDEL is retained in the endoplasmic reticulum and accumulates to high levels in the leaves of transgenic plants. Plant J. 2: 181-192.
Yabuta, M., Suzuki, Y. and Ohsuye, K. 1995. High expression of a recombinant human calcitonin precursor peptide in Escherichia coli. Appl. Microbiol. Biotechnol. 42 (5): 703-708.
Yamamoto, K., Fujii, R., Toyofuku, Y., Saito, T., Koseki, H., Hsu V.W. and Aoe, T. 2001. The KDEL receptor mediates a retrieval machanism that contributes to quality control at the endoplasmic reticulum. EMBO J. 20 (12): 3082-3091.

## Claims

1. A nucleic acid sequence comprising:
a first nucleic acid sequence containing the nucleotide sequence that encodes the protein γ-zein, or a fragment thereof containing a nucleotide sequence that encodes an amino acid sequence capable of directing and retaining a protein towards the endoplasmic reticulum (ER) of a plant cell;
a second nucleic acid sequence containing a nucleotide sequence that encodes an amino acid sequence that is specifically cleavable by enzymatic or chemical means; and
a third nucleic acid sequence containing the nucleotide sequence that encodes a product of interest;
wherein the 3' end of said first nucleic acid sequence is linked to the 5' end of said second nucleic acid sequence and the 3' end of said second nucleic acid sequence is linked to the 5' end of said third nucleic acid sequence.

2. Nucleic acid sequence according to claim 1, wherein said first nucleic acid sequence comprises a nucleotide sequence that encodes the full-length γ-zein protein.

3. Nucleic acid sequence according to claim 1, wherein said first nucleic acid sequence comprises:
- one or more nucleotide sequences encoding all or part of the repetition domain of the protein γ-zein;
- one or more nucleotide sequences encoding all or part of the ProX domain of the protein γ-zein; or
- one or more nucleotide sequence encoding all or part of the repetition domain of the protein γ-zein, and one or more nucleotide sequences encoding all or part of the ProX domain of the protein γ-zein.

4. Nucleic acid sequence according to claim 1, wherein said first nucleic acid sequence is chosen from the following group:
- the nucleotide sequence shown in SEQ ID NO: 1 [nucleotide sequence that encodes γ-zein (Figure 1A)],
- the nucleotide sequence shown in SEQ ID NO: 2 [nucleotide sequence identified as RX3 (Figure 1B)],
- the nucleotide sequence shown in SEQ ID NO: 3 [nucleotide sequence identified as R3 (Figure 1B)],
- the nucleotide sequence shown in SEQ ID NO: 4 [nucleotide sequence identified as P4 (Figure 1C)], and
- the nucleotide sequence shown in SEQ ID NO: 5 [nucleotide sequence identified as X10 (Figure 1C)].

5. Nucleic acid sequence according to claims 1-4, wherein said second nucleic acid sequence comprises a nucleotide sequence that encodes an amino acid sequence that defines a protease cleavage site.

6. Nucleic acid sequence according to claim 5, wherein said protease is an enterokinase, an Arg-C endoprotease, a Glu-C endoprotease, a Lys-C endoprotease or factor Xa.

7. Nucleic acid sequence according to claims 1-4, wherein said second nucleic acid sequence comprises a nucleotide sequence that encodes an amino acid that is specifically cleavable by a chemical reagent.

8. Nucleic acid sequence according to claim 7, wherein said chemical reagent is cyanogen bromide.

9. Nucleic acid sequence according to claims 1-8, wherein said product of interest is a proteinaceous drug.

10. Nucleic acid sequence according to claim 9, wherein said proteinaceous drug is a peptide hormone or an interferon, said drug being effective for treating the human or animal body.

11. Nucleic acid sequence according to claim 10, wherein said peptide hormone is selected from calcitonin, erythropoietin, thrombopoietin and growth hormone.

12. Nucleic acid sequence according to claim 1, wherein said third nucleic acid sequence comprises a nucleotide sequence encoding calcitonin and a codon for glycine at the 3' end of said nucleic acid sequence encoding calcitonin.

13. A fusion protein comprising:
(i) the amino acid sequence of the protein γ-zein, or a fragment thereof capable of directing and retaining a protein towards the ER of a plant cell,
(ii) an amino acid sequence that is specifically cleavable by enzymatic or chemical means, and
(iii) a product of interest;
said fusion protein being the expression product of the nucleic acid sequence of any of claims 1-12 in a host plant system.

14. Fusion protein according to claim 13, comprising a full-length γ-zein protein.

15. Fusion protein according to claim 14, comprising the amino acid sequence shown in Figure 1A and identified in SEQ ID NO: 6.

16. Fusion protein according to claim 13, comprising a fragment of a γ-zein protein, said fragment containing an amino acid sequence capable of directing and retaining a protein towards the ER of a plant cell.

17. Fusion protein according to claim 16, comprising a fragment of a γ-zein protein selected from the group consisting of:
- the amino acid sequence shown in SEQ ID NO: 7 [amino acid sequence corresponding to RX3 (Figure 1B)],
- the amino acid sequence shown in SEQ ID NO: 8 [amino acid sequence corresponding to R3 (Figure 1B)],
- the amino acid sequence shown in SEQ ID NO: 9 [amino acid sequence corresponding to P4 (Figure 1C)], and
- the amino acid sequence shown in SEQ ID NO: 10 [amino acid sequence corresponding to X10 (Figure 1C)].

18. Fusion protein according to claim 13, wherein said amino acid sequence that is specifically cleavable by enzymatic means comprises a protease cleavage site.

19. Fusion protein according to claim 13, wherein said amino acid sequence that is specifically cleavable by chemical means comprises a cleavage site cleavable by a chemical reagent.

20. Fusion protein according to claim 13, wherein said product of interest is a proteinaceous drug.

21. A nucleic acid construct comprising (i) a nucleic acid sequence according to any of claims 1 to 12, and (ii) a regulatory nucleotide sequence that regulates the transcription of the nucleic acid of the invention (i), said regulatory sequence (ii) being functional in plants.

22. Construct according to claim 21, wherein said regulatory sequence (ii) is tissue-specific.

23. Construct according to claim 21, wherein said regulatory sequence (ii) comprise a promoter functional in plants.

24. Construct according to claim 22, wherein said regulatory sequence (ii) comprises the promoter 35SCaMV, the "patatina" promoter, a storage protein promoter, the ubiquitine gene promoter or the regulatory sequences of the γ-zein gene.

25. Construct according to claim 21, wherein said regulatory sequence (ii) comprises a transcription termination sequence functional in plants.

26. Construct according to claim 25, wherein said regulatory sequence (ii) comprises the terminator 35SCaMV, the terminator of the octopine synthase (ocs) gene, the terminator of the nopaline synthase (nos) gene or the terminator of the γ-zein gene.

27. Construct according to claim 21, wherein said regulatory sequence (ii) further comprises a translation enhancer functional in plant.

28. Construct according to claim 27, wherein said translation enhancer functional in plant comprises the promoting sequence for transcription of the tomato etch virus, and the like.

29. A vector comprising a nucleic acid sequence according to any of claims 1 to 12, or a nucleic acid construct according to any of claims 21 to 28.

30. A transformed plant host system, said plant host system having been transformed with a nucleic acid sequence according to any of claims 1 to 12, or with a nucleic acid construct according to any of claims 21 to 28, or with a vector according to claim 29.

31. A transgenic plant host system, said transgenic plant host system comprising, integrated in its genome, a nucleic acid sequence according to any of claims 1 to 13.

32. Plant host system according to claim 30 or 31, wherein said plant host system is a monocot or a dicot plant.

33. Plant host system according to claim 32, wherein said plant host system is a cereal, a legume, a cruciferous or a solanaceous.

34. Plant host system according to claim 30 or 31, comprising a seed.

35. A method for producing a product of interest in a plant host system, which comprises growing a transformed or transgenic plant host system according to claims 30-34, under conditions that allow the production and expression of said product of interest in the form of a fusion protein.

36. Method according to claim 35, which further comprises the isolation and purification of said fusion protein.

37. Method according to claim 35 or 36, which further comprises the release of said product of interest from said fusion protein.

38. A method for producing calcitonin in a plant host system, comprising:
a) transforming a plant host system with an expression vector or with a nucleic acid construct, comprising a regulatory sequence for the transcription of a nucleic acid sequence (nucleic acid sequence of the invention) that consists of:
a first nucleic acid sequence containing the nucleotide sequence that encodes the protein γ-zein, or a fragment thereof containing a nucleotide sequence that encodes an amino acid sequence capable of directing and retaining a protein towards the endoplasmic reticulum (ER) of a plant cell;
a second nucleic acid sequence containing a nucleotide sequence that encodes an amino acid sequence that is specifically cleavable by enzymatic or chemical means; and
a third nucleic acid sequence containing the nucleotide sequence that encodes calcitonin;
wherein the 3' end of said first nucleic acid sequence is linked to the 5' end of said second nucleic acid sequence and the 3' end of said second nucleic acid sequence is linked to the 5' end of said third nucleic acid sequence;
b) generating complete plants from said plant host systems transformed with said expression vector or nucleic acid construct;
c) growing such transformed plants under conditions that allow the production and expression of calcitonin in the form of a fusion protein; and, if desired
d) isolating, purifying said fusion protein and treating said fusion protein in order to release calcitonin.

## Patentansprüche

1. Nukleinsäuresequenz, umfassend:
eine erste Nukleinsäuresequenz, enthaltend die Nukleotidsequenz, welche das Protein γ-zein kodiert oder ein Fragment davon, enthaltend eine Nukleotidsequenz, welche eine Aminosäuresequenz kodiert, die befähigt ist, ein Protein zum endoplasmatischen Reticulum (ER) einer Pflanzenzelle zu dirigieren und zurückzubehalten,
eine zweite Nukleinsäuresequenz, enthaltend eine Nukleotidsequenz, welche eine Aminosäuresequenz kodiert, die spezifisch durch enzymatische oder chemische Mittel spaltbar ist; und
eine dritte Nukleinsäuresequenz, enthaltend die Nukleotidsequenz, welche ein Produkt von Interesse kodiert;
wobei das 3'-Ende der ersten Nukleinsäuresequenz mit dem 5'-Ende der zweiten Nukleinsäuresequenz verbunden ist, und das 3'-Ende der zweiten Nukleinsäuresequenz mit dem 5'-Ende der dritten Nukleinsäuresequenz verbunden ist.

2. Nukleinsäuresequenz nach Anspruch 1, wobei die erste Nukleinsäuresequenz eine Nukleotidsequenz umfaßt, welche das γ-zein-Protein in voller Länge kodiert.

3. Nukleinsäuresequenz nach Anspruch 1, wobei die erste Nukleinsäuresequenz umfaßt:
- eine oder mehrere Nukleotidsequenz(en), welche alle oder einen Teil der Wiederholungsdomäne des Proteins γ-zein kodieren,
- eine oder mehrere Nukleotidsequenz(en), welche alle oder einen Teil der ProX-Domäne des Proteins γ-zein kodieren, oder
- eine oder mehrere Nukleotidsequenz(en), welche alle oder einen Teil der Wiederholungsdomäne des Proteins γ-zein kodieren, und eine oder mehrere Nukleotidsequenz(en), welche alle oder einen Teil der ProX-Domäne des Proteins γ-zein kodieren.

4. Nukleinsäuresequenz nach Anspruch 1, wobei die erste Nukleinsäuresequenz aus der folgenden Gruppe ausgewählt ist:
- die in SEQ ID Nr. 1 gezeigte Nukleotidsequenz [Nukleotidsequenz, die γ-zein kodiert (Fig. 1A)],
- die in SEQ ID Nr. 2 gezeigte Nukleotidsequenz [als RX3 identifizierte Nukleotidsequenz (Fig. 1 B)],
- die in SEQ ID Nr. 3 gezeigte Nukleotidsequenz [als R3 identifizierte Nukleotidsequenz (Fig. 1 B)],
- die in SEQ ID Nr. 4 gezeigte Nukleotidsequenz [als P4 identifizierte Nukleotidsequenz (Fig. 1 C)], und
- die in SEQ ID Nr. 5 gezeigte Nukleotidsequenz [als X10 identifizierte Nukleotidsequenz (Fig. 1C)].

5. Nukleinsäuresequenz nach einem der Ansprüche 1 bis 4, wobei die zweite Nukleinsäuresequenz eine Nukleotidsequenz umfaßt, welche eine Aminosäuresequenz kodiert, die eine Proteasespaltstelle definiert.

6. Nukleinsäuresequenz nach Anspruch 5, wobei die Protease eine Enterokinase, eine Arg-C-Endoprotease, eine Glu-C-Endoprotease, eine Lys-C-Endoprotease oder Faktor Xa ist.

7. Nukleinsäuresequenz nach einem der Ansprüche 1-4, wobei die zweite Nukleinsäuresequenz eine Nukleotidsequenz umfaßt, welche eine Aminosäure kodiert, die spezifisch durch ein chemisches Reagens spaltbar ist.

8. Nukleinsäuresequenz nach Anspruch 7, wobei das chemische Reagens Cyanbromid ist.

9. Nukleinsäuresequenz nach einem der Ansprüche 1-8, wobei das Produkt von Interesse ein proteinartiges Arzneimittel ist.

10. Nukleinsäuresequenz nach Anspruch 9, wobei das proteinartige Arzneimittel ein Peptidhormon oder ein Interferon ist, wobei das Arzneimittel wirksam ist, den menschlichen oder tierischen Körper zu behandeln.

11. Nukleinsäuresequenz nach Anspruch 10, wobei das Peptidhormon ausgewählt ist aus Calcitonin, Erythropoietin, Thrombopoietin und Wachstumshormon.

12. Nukleinsäuresequenz nach Anspruch 1, wobei die dritte Nukleinsäuresequenz eine Nukleotidsequenz umfaßt, welche Calcitonin und ein Codon für Glycin am 3'-Ende der Calcitonin-kodierenden Nukleinsäuresequenz kodiert.

13. Fusionsprotein, umfassend
(i) die Aminosäuresequenz des Proteins γ-zein oder ein Fragment davon, welches befähigt ist, ein Protein zu dem ER einer Pflanzenzelle zu dirigieren und zurückzuhalten,
(ii) eine Aminosäuresequenz, welche spezifisch durch ein enzymatisches oder chemisches Mittel spaltbar ist, und
(iii) ein Produkt von Interesse,
wobei das Fusionsprotein das Expressionsprodukt der Nukleinsäuresequenz nach einem der Ansprüche 1-12 in einem Wirtspflanzensystem ist.

14. Fusionsprotein nach Anspruch 13, umfassend ein γ-zein-Protein in voller Länge.

15. Fusionsprotein nach Anspruch 14, umfassend die in Fig. 1A gezeigte und in SEQ ID Nr. 6 identifizierte Aminosäuresequenz.

16. Fusionsprotein nach Anspruch 13, umfassend ein Fragment eines γ-zein-Proteins, wobei das Fragment eine Aminosäuresequenz enthält, welche befähigt ist, ein Protein zu dem ER einer Pflanzenzelle zu dirigieren und zurückzubehalten.

17. Fusionsprotein nach Anspruch 16, umfassend ein Fragment eines γ-zein-Proteins, ausgewählt aus der Gruppe, bestehend aus:
- der in SEQ ID Nr. 7 gezeigten Aminosäuresequenz [Aminosäuresequenz, welche RX3 entspricht (Fig. 1 B)],
- der in SEQ ID Nr. 8 gezeigten Aminosäuresequenz [Aminosäuresequenz, welche R3 entspricht (Fig. 1 B)],
- der in SEQ ID Nr. 9 gezeigten Aminosäuresequenz [Aminosäuresequenz, welche P4 entspricht (Fig. 1 C)], und
- der in SEQ ID Nr. 10 gezeigten Aminosäuresequenz [Aminosäuresequenz, welche X10 entspricht (Fig. 1C)].

18. Fusionsprotein nach Anspruch 13, wobei die Aminosäuresequenz, welche durch ein enzymatisches Mittel spezifisch spaltbar ist, eine Proteasespaltstelle umfaßt.

19. Fusionsprotein nach Anspruch 13, wobei die Aminosäuresequenz, welche spezifisch durch ein chemisches Mittel spaltbar ist, eine durch ein chemisches Reagens spaltbare Stelle umfaßt.

20. Fusionsprotein nach Anspruch 13, wobei das Produkt von Interesse ein proteinartiges Arzneimittel ist.

21. Nukleinsäurekonstrukt, umfassend (i) eine Nukleinsäuresequenz nach einem der Ansprüche 1 bis 12 und (ii) eine regulatorische Nukleotidsequenz, welche die Transkription der Nukleinsäure der Erfindung (i) reguliert, wobei die regulatorische Sequenz (ii) funktionsfähig in Pflanzen ist.

22. Konstrukt nach Anspruch 21, wobei die regulatorische Sequenz (ii) gewebespezifisch ist.

23. Konstrukt nach Anspruch 21, wobei die regulatorische Sequenz (ii) einen in Pflanzen funktionsfähigen Promotor umfaßt.

24. Konstrukt nach Anspruch 22, wobei die regulatorische Sequenz (ii) den Promotor 35SCaMV, den "Patatina"-Promotor, einen Speicherproteinpromotor, den Ubiquitingenpromotor oder die regulatorischen Sequenzen des γ-zein-Gens umfaßt.

25. Konstrukt nach Anspruch 21, wobei die regulatorische Sequenz (ii) eine in Pflanzen funktionsfähige Transkriptions-Terminationssequenz umfaßt.

26. Konstrukt nach Anspruch 25, wobei die regulatorische Sequenz (ii) den Terminator 35SCaMV, den Terminator des Octopinsynthase(ocs)-Gens, den Terminator des Nopalinsynthase(nos)-Gens oder den Terminator des γ-zein-Gens umfaßt.

27. Konstrukt nach Anspruch 21, wobei die regulatorische Sequenz (ii) weiter einen in Pflanzen funktionsfähigen Translationsverstärker umfaßt.

28. Konstrukt nach Anspruch 27, wobei der in einer Pflanze funktionsfähige Translationsverstärker die Promotionssequenz zur Transkription des Tomatenätzvirus und ähnlichem, umfaßt.

29. Vektor, umfassend eine Nukleinsäuresequenz nach einem der Ansprüche 1 bis 12 oder ein Nukleinsäurekonstrukt nach einem der Ansprüche 21 bis 28.

30. Transformiertes Pflanzenwirtssystem, wobei das Pflanzenwirtssystem mit einer Nukleinsäuresequenz nach einem der Ansprüche 1 bis 12 oder mit einem Nukleinsäurekonstrukt nach einem der Ansprüche 21 bis 28 oder mit einem Vektor nach Anspruch 29 transformiert ist.

31. Transgenes Pflanzenwirtssystem, wobei das transgene Pflanzenwirtssystem, in sein Genom integriert, eine Nukleinsäuresequenz nach einem der Ansprüche 1 bis 12 umfaßt.

32. Pflanzenwirtssystem nach Anspruch 30 oder 31, wobei das Pflanzenwirtssystem eine einkeimblättrige oder eine zweikeimblättrige Pflanze ist.

33. Pflanzenwirtssystem nach Anspruch 32, wobei das Pflanzenwirtssystem ein Getreide, eine Frucht bzw. Legume, ein Kreuzblütler oder ein Nachtschattengewächs ist.

34. Pflanzenwirtssystem nach Anspruch 30 oder 31, umfassend einen Samen.

35. Verfahren zur Herstellung eines Produkts von Interesse in einem Pflanzenwirtssystem, welches das Züchten eines transformierten oder transgenischen Pflanzenwirtssystems nach Anspruch 30 bis 34 umfaßt, unter Bedingungen, welche die Herstellung und Expression des Produkts von Interesse in der Form eines Fusionsproteins erlauben.

36. Verfahren nach Anspruch 35, welches weiter die Isolation und Reinigung des Fusionsproteins umfaßt.

37. Verfahren nach Anspruch 35 oder 36, welches weiter die Freisetzung des Produkts von Interesse von dem Fusionsprotein umfaßt.

38. Verfahren zur Herstellung von Calcitonin in einem Pflanzenwirtssystem, umfassend:
a) das Transformieren eines Pflanzenwirtssystems mit einem Expressionsvektor oder mit einem Nukleinsäurekonstrukt, umfassend eine regulatorische Sequenz zur Transkription einer Nukleinsäuresequenz (Nukleinsäuresequenz der Erfindung), welche besteht aus:
einer ersten Nukleinsäuresequenz, enthaltend die Nukleotidsequenz, die das Protein γ-zein kodiert oder ein Fragment davon, enthaltend eine Nukleotidsequenz, die eine Aminosäuresequenz kodiert, welche befähigt ist, ein Protein zu dem endoplasmatischen Reticulum (ER) einer Pflanzenzelle zu dirigieren und zurückzuhalten,
einer zweiten Nukleinsäuresequenz, enthaltend eine Nukleotidsequenz, welche eine Aminosäuresequenz kodiert, die spezifisch durch enzymatische oder chemische Mittel spaltbar ist, und
einer dritten Nukleinsäuresequenz, enthaltend die Nukleotidsequenz, welche Calcitonin kodiert,
wobei das 3'-Ende der ersten Nukleinsäuresequenz mit dem 5'-Ende der zweiten Nukleinsäuresequenz verbunden ist, und das 3'-Ende der zweiten Nukleinsäuresequenz mit dem 5'-Ende der dritten Nukleinsäuresequenz verbunden ist,
b) das Erzeugen vollständiger Pflanzen von dem Pflanzenwirtssystem, welches mit dem Expressionsvektor oder dem Nukleinsäurekonstrukt transformiert ist,
c) das Züchten solch transformierter Pflanzen unter Bedingungen, die die Herstellung und Expression von Calcitonin in der Form eines Fusionsproteins erlauben, und, falls gewünscht,
d) das Isolieren, Reinigen des Fusionsproteins, und das Behandeln des Fusionsproteins, um Calcitonin freizusetzen.

## Revendications

1. Séquence d'acide nucléique comprenant :
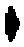 une première séquence d'acide nucléique contenant la séquence nucléotidique qui code pour la protéine γ-zein, ou un fragment de celle-ci contenant une séquence nucléotidique qui code pour une séquence d'acides aminés capable de diriger et de retenir une protéine dans le réticulum endoplasmique (RE) d'une cellule de plante ;
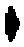 une seconde séquence d'acide nucléique contenant une séquence nucléotidique qui code pour une séquence d'acides aminés qui est spécifiquement clivable par un moyen enzymatique ou chimique ; et
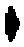 une troisième séquence d'acide nucléique contenant la séquence nucléotidique qui code pour un produit d'intérêt ;
dans laquelle l'extrémité 3' de ladite première séquence d'acide nucléique est liée à l'extrémité 5' de ladite seconde séquence d'acide nucléique, et l'extrémité 3' de ladite seconde séquence d'acide nucléique est liée à l'extrémité 5' de ladite troisième séquence d'acide nucléique.

2. Séquence d'acide nucléique selon la revendication 1, dans laquelle ladite première séquence d'acide nucléique comprend une séquence nucléotidique qui code pour la protéine γ-zein de pleine longueur.

3. Séquence d'acide nucléique selon la revendication 1, dans laquelle ladite première séquence d'acide nucléique comprend :
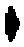 une ou plusieurs séquences nucléotidiques codant pour tout ou partie du domaine de répétition de la protéine γ-zein ;
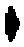 une ou plusieurs séquences nucléotidiques codant pour tout ou partie du domaine ProX de la protéine γ-zein ; ou
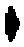 une ou plusieurs séquences nucléotidiques codant pour tout ou partie du domaine de répétition de la protéine γ-zein, et une ou plusieurs séquences nucléotidiques codant pour tout ou partie du domaine ProX de la protéine γ-zein.

4. Séquence d'acide nucléique selon la revendication 1, dans laquelle ladite première séquence d'acide nucléique est choisie dans le groupe suivant :
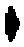 la séquence nucléotidique représentée dans SEQ ID n° : 1 [séquence nucléotidique qui code pour la protéine γ-zein (figure 1A)],
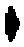 la séquence nucléotidique représentée dans SEQ ID n° : 2 [séquence nucléotidique identifiée en tant que RX3 (figure 1B)],
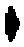 la séquence nucléotidique représentée dans SEQ ID n° : 3 [séquence nucléotidique identifiée en tant que R3 (figure 1B)],
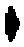 la séquence nucléotidique représentée dans SEQ ID n° : 4 [séquence nucléotidique identifiée en tant que P4 (figure 1C)] et
la séquence nucléotidique représentée dans SEQ ID n° : 5 [séquence nucléotidique identifiée en tant que X10 (figure 1C)].

5. Séquence d'acide nucléique selon les revendications 1 à 4, dans laquelle ladite seconde séquence d'acide nucléique comprend une séquence nucléotidique qui code pour une séquence d'acides aminés qui définit un site de clivage par une protéase.

6. Séquence d'acide nucléique selon la revendication 5, dans laquelle ladite protéase est une entérokinase, une Arg-C endoprotéase, une Glu-C endoprotéase, une Lys-C endoprotéase ou un facteur Xa.

7. Séquence d'acide nucléique selon les revendications 1 à 4, dans laquelle ladite seconde séquence d'acide nucléique comprend une séquence nucléotidique qui code pour un acide aminé qui est spécifiquement clivable par un réactif chimique.

8. Séquence d'acide nucléique selon la revendication 7, dans laquelle ledit réactif chimique est le bromure de cyanogène.

9. Séquence d'acide nucléique selon les revendications 1 à 8, dans laquelle ledit produit d'intérêt est un médicament protéique.

10. Séquence d'acide nucléique selon la revendication 9, dans laquelle ledit médicament protéique est une hormone peptidique ou un interféron, ledit médicament étant efficace pour le traitement du corps humain ou animal.

11. Séquence d'acide nucléique selon la revendication 10, dans laquelle ladite hormone peptidique est choisie parmi la calcitonine, l'érythropoïétine, la thrombopoïétine et une hormone de croissance.

12. Séquence d'acide nucléique selon la revendication 1, dans laquelle ladite troisième séquence d'acide nucléique comprend une séquence nucléotidique codant pour la calcitonine et un codon pour la glycine à l'extrémité 3' de ladite séquence d'acide nucléique codant pour la calcitonine.

13. Protéine de fusion comprenant :
(i) la séquence d'acides aminés de la protéine γ-zein ou un fragment de celle-ci capable de diriger et de retenir une protéine dans le RE d'une cellule de plante,
(ii) une séquence d'acides aminés qui est spécifiquement clivable par un moyen enzymatique ou chimique, et
(iii) un produit d'intérêt ;
ladite protéine de fusion étant le produit d'expression de la séquence d'acide nucléique selon l'une quelconque des revendications 1 à 12 dans un système de plante hôte.

14. Protéine de fusion selon la revendication 13, comprenant la protéine γ-zein de pleine longueur.

15. Protéine de fusion selon la revendication 14, comprenant la séquence d'acides animés représentée sur la figure 1A et identifiée en tant que SEQ ID n° : 6.

16. Protéine de fusion selon la revendication 13, comprenant un fragment d'une protéine γ-zein, ledit fragment contenant une séquence d'acides aminés capable de diriger et de retenir une protéine dans le RE d'une cellule de plante.

17. Protéine de fusion selon la revendication 16, comprenant un fragment d'une protéine γ-zein choisi dans le groupe constitué de :
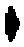 la séquence d'acides aminés représentée dans SEQ ID n° : 7 [séquence d'acides aminés correspondant à RX3 (figure 1B)],
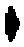 la séquence d'acides aminés représentée dans SEQ ID n° : 8 [séquence d'acides aminés correspondant à R3 (figure 1B)],
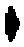 la séquence d'acides aminés représentée dans SEQ ID n° : 9 [séquence d'acides aminés correspondant à P4 (figure 1C)] et
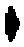 la séquence d'acides aminés représentée dans SEQ ID n° : 10 [séquence d'acides aminés correspondant à X10 (figure 1C)].

18. Protéine de fusion selon la revendication 13, dans laquelle ladite séquence d'acides aminés qui est spécifiquement clivable par un moyen enzymatique comprend un site de clivage à une protéase.

19. Protéine de fusion selon la revendication 13, dans laquelle ladite séquence d'acides aminés qui est spécifiquement clivable par un moyen chimique comprend un site de clivage clivable par un réactif chimique.

20. Protéine de fusion selon la revendication 13, dans laquelle ledit produit d'intérêt est un médicament protéique.

21. Construction d'acide nucléique comprenant (i) une séquence d'acide nucléique selon l'une quelconque des revendications 1 à 12 et (ii) une séquence nucléotidique régulatrice qui régule la transcription de l'acide nucléique de l'invention (i), ladite séquence régulatrice (ii) étant fonctionnelle chez les plantes.

22. Construction selon la revendication 21, dans laquelle ladite séquence régulatrice (ii) est tissu-spécifique.

23. Construction selon la revendication 21, dans laquelle ladite séquence régulatrice (ii) comprend un promoteur fonctionnel chez les plantes.

24. Construction selon la revendication 22, dans laquelle ladite séquence régulatrice (ii) comprend le promoteur 35SCaMV, le promoteur "patatina", un promoteur de stockage protéique, le promoteur du gène de l'ubiquitine ou les séquences régulatrices du gène γ-zein.

25. Construction selon la revendication 21, dans laquelle ladite séquence régulatrice (ii) comprend une séquence de terminaison de la transcription fonctionnelle chez les plantes.

26. Construction selon la revendication 25, dans laquelle ladite séquence régulatrice (ii) comprend la séquence de terminaison 35SCaMV, la séquence de terminaison du gène de l'octopine synthétase (ocs), la séquence de terminaison du gène de la nopaline synthétase (nos) ou la séquence de terminaison du gène de la γ-zein.

27. Construction selon la revendication 21, dans laquelle ladite séquence régulatrice (ii) comprend en outre un amplificateur de traduction fonctionnel chez une plante.

28. Construction selon la revendication 27, dans laquelle ledit amplificateur de traduction fonctionnel chez une plante comprend la séquence promotrice pour la transcription du virus de la rainure de la tomate et analogue.

29. Vecteur comprenant une séquence d'acide nucléique selon l'une quelconque des revendications 1 à 12, ou une construction d'acide nucléique selon l'une quelconque des revendications 21 à 28.

30. Système hôte de plante transformée, ledit système hôte de plante ayant été transformé par une séquence d'acide nucléique selon l'une quelconque des revendications 1 à 12 ou par une construction d'acide nucléique selon l'une quelconque des revendications 21 à 28, ou par un vecteur selon la revendication 29.

31. Système hôte de plante transgénique, ledit système hôte de plante transgénique comprenant, intégré dans son génome, une séquence d'acide nucléique selon l'une quelconque des revendications 1 à 12.

32. Système hôte de plante selon la revendication 30 ou 31, dans lequel ledit système hôte de plante est une plante monocotylédone ou dicotylédone.

33. Système hôte de plante selon la revendication 32, dans lequel ledit système hôte de plante est une céréale, un légume, une crucifère ou une solanacée.

34. Système hôte de plante selon la revendication 30 ou 31, comprenant une graine.

35. Procédé pour la production d'un produit d'intérêt dans un système hôte de plante, qui comprend la croissance d'un système hôte de plante transformée ou transgénique selon les revendications 30 à 34, dans des conditions qui permettent la production et l'expression dudit produit d'intérêt sous la forme d'une protéine de fusion.

36. Procédé selon la revendication 35, qui comprend en outre l'isolement et la purification de ladite protéine de fusion.

37. Procédé selon la revendication 35 ou 36, qui comprend en outre la libération dudit produit d'intérêt à partir de ladite protéine de fusion.

38. Procédé pour la production de calcitonine dans un système hôte de plante, comprenant :
a) la transformation d'un système hôte de plante par un vecteur d'expression ou par une construction d'acide nucléique, comprenant une séquence régulatrice pour la transcription d'une séquence d'acide nucléique (séquence d'acide nucléique de l'invention) qui est constituée de :
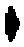 une première séquence d'acide nucléique contenant la séquence nucléotidique qui code pour la protéine γ-zein, ou un fragment de celle-ci contenant une séquence nucléotidique qui code pour une séquence d'acides aminés capable de diriger et de retenir une protéine dans le réticulum endoplasmique (RE) d'une cellule de plante ;
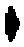 une seconde séquence d'acide nucléique contenant une séquence nucléotidique qui code pour une séquence d'acides aminés qui est spécifiquement clivable par un moyen enzymatique ou chimique ; et
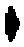 une troisième séquence d'acide nucléique contenant la séquence nucléotidique qui code pour la calcitonine ;
dans laquelle l'extrémité 3' de ladite première séquence d'acide nucléique est liée à l'extrémité 5' de ladite seconde séquence d'acide nucléique, et l'extrémité 3' de ladite seconde séquence d'acide nucléique est liée à l'extrémité 5' de ladite troisième séquence d'acide nucléique ;
b) la création de plantes complètes à partir desdits systèmes hôtes de plante transformée par ledit vecteur d'expression ou ladite construction d'acide nucléique ;
c) la croissance de telles plantes transformées dans des conditions qui permettent la production et l'expression de la calcitonine sous la forme d'une protéine de fusion ; et, si on le désire
d) l'isolement, la purification de ladite protéine de fusion et le traitement de ladite protéine de fusion afin de libérer la calcitonine.


### SEQUENCE LISTING

<110> Advanced in vitro Cell Technologies, S.L.
<120> Production of products of interest by accumulation in endoplasmic reticulum-derived protein bodies
<160> 10
<210> 1
   <211> 672
   <212> DNA
   <213> Zea mays
<400> 1
   atgagggtgt tgctcgttgc cctcgctctc ctggctctcg ctgcgagcgc cacctccacg   60
   catacaagcg gcggctgcgg ctgccagcca ccgccgccgg ttcatctacc gccgccggtg  120
   catctgccac ctccggttca cctgccacct ccggtgcatc tcccaccgcc ggtccacctg  180
   ccgccgccgg tccacctgcc accgccggtc catgtgccgc cgccggttca tctgccgccg  240
   ccaccatgcc actaccctac tcaaccgccc cggcctcagc ctcatcccca gccacaccca  300
   tgcccgtgcc aacagccgca tccaagcccg tgccagctgc agggaacctg cggcgttggc  360
   agcaccccga tcctgggcca gtgcgtcgag tttctgaggc atcagtgcag cccgacggcg  420
   acgccctact gctcgcctca gtgccagtcg ttgcggcagc agtgttgcca gcagctcagg  480
   caggtggagc cgcagcaccg gtaccaggcg atcttcggct tggtcctcca gtccatcctg  540
   cagcagcagc cgcaaagcgg ccaggtcgcg gggctgttgg cggcgcagat agcgcagcaa  600
   ctgacggcga tgtgcggcct gcagcagccg actccatgcc cctacgctgc tgccggcggt  660
   gtcccccacg cc                                                      672
<210> 2
   <211> 339
   <212> DNA
   <213>
<400> 2
   atgagggtgt tgctcgttgc cctcgctctc ctggctctcg ctgcgagcgc cacctccacg   60
   catacaagcg gcggctgcgg ctgccagcca ccgccgccgg ttcatctacc gccgccggtg  120
   catctgccac ctccggttca cctgccacct ccggtgcatc tcccaccgcc ggtccacctg  180
   ccgccgccgg tccacctgcc accgccggtc catgtgccgc cgccggttca tctgccgccg  240
   ccaccatgcc actaccctac tcaaccgccc cggcctcagc ctcatcccca gccacaccca  300
   tgcccgtgcc aacagccgca tccaagcccg tgccagacc                         339
<210> 3
   <211> 276
   <212> DNA
   <213>
<400> 3
   atgagggtgt tgctcgttgc cctcgctctc ctggctctcg ctgcgagcgc cacctccacg   60
   catacaagcg gcggctgcgg ctgccagcca ccgccgccgg ttcatctacc gccgccggtg  120
   catctgccac ctccggttca cctgccacct ccggtgcatc tcccaccgcc ggtccacctg  180
   ccgccgccgg tccacctgcc accgccggtc catgtgccgc cgccggttca tctgccgccg  240
   ccaccatgcc actaccctac tcaaccgccc cggacc                            276
<210> 4
   <211> 213
   <212> DNA
   <213>
<400> 4
   atgagggtgt tgctcgttgc cctcgctctc ctggctctcg ctgcgagcgc cacctccacg   60
   catacaagcg gcggctgcgg ctgccagcca ccgccgccgg ttcatctgcc gccgccacca  120
   tgccactacc ctacacaacc gccccggcct cagcctcatc cccagccaca cccatgcccg  180
   tgccaacagc cgcatccaag cccgtgccag acc                               213
<210> 5
   <211> 180
   <212> DNA
   <213>
<400> 5
   atgagggtgt tgctcgttgc cctcgctctc ctggctctcg ctgcgagcgc cacctccacg   60
   catacaagcg gcggctgcgg ctgccaatgc cactacccta ctcaaccgcc ccggcctcag  120
   cctcatcccc agccacaccc atgcccgtgc caacagccgc atccaagccc gtgccagacc  180
<210> 6
   <211> 224
   <212> PRT
   <213> Zea mays
<400> 6
   Met Arg Val Leu Leu Val Ala Leu Ala Leu Leu Ala Leu Ala Ala Ser
    1               5                  10                  15
   Ala Thr Ser Thr His Thr Ser Gly Gly Cys Gly Cys Gln Pro Pro Pro
               20                  25                  30
   Pro Val His Leu Pro Pro Pro Val His Leu Pro Pro Pro Val His Leu
           35                  40                  45
   Pro Pro Pro Val His Leu Pro Pro Pro Val His Leu Pro Pro Pro Val
       50                  55                  60
   His Leu Pro Pro Pro Val His Val Pro Pro Pro Val His Leu Pro Pro
   65                  70                  75                  80
   Pro Pro Cys His Tyr Pro Thr Gln Pro Pro Arg Pro Gln Pro His Pro
                   85                  90                  95
   Gln Pro His Pro Cys Pro Cys Gln Gln Pro His Pro Ser Pro Cys Gln
               100                 105                 110
   Leu Gln Gly Thr Cys Gly Val Gly Ser Thr Pro Ile Leu Gly Gln Cys
           115                 120                 125
   Val Glu Phe Leu Arg His Gln Cys Ser Pro Thr Ala Thr Pro Tyr Cys
       130                 135                 140
   Ser Pro Gln Cys Gln Ser Leu Arg Gln Gln Cys Cys Gln Gln Leu Arg
   145                 150                 155                 160
   Gln Val Glu Pro Gln His Arg Tyr Gln Ala Ile Phe Gly Leu Val Leu
                   165                 170                 175
   Gln Ser Ile Leu Gln Gln Gln Pro Gln Ser Gly Gln Val Ala Gly Leu
               180                 185                 190
   Leu Ala Ala Gln Ile Ala Gln Gln Leu Thr Ala Met Cys Gly Leu Gln
           195                 200                 205
   Gln Pro Thr Pro Cys Pro Tyr Ala Ala Ala Gly Gly Val Pro His Ala
       210                 215                 220
<210> 7
   <211> 113
   <212> PRT
<400> 7
   Met Arg Val Leu Leu Val Ala Leu Ala Leu Leu Ala Leu Ala Ala Ser
   1 5 10 15
   Ala Thr Ser Thr His Thr Ser Gly Gly Cys Gly Cys Gln Pro Pro Pro
   20 25 30
   Pro Val His Leu Pro Pro Pro Val His Leu Pro Pro Pro Val His Leu
   35 40 45
   Pro Pro Pro Val His Leu Pro Pro Pro Val His Leu Pro Pro Pro Val
   50 55 60
   His Leu Pro Pro Pro Val His Val Pro Pro Pro Val His Leu Pro Pro
   65 70 75 80
   Pro Pro Cys His Tyr Pro Thr Gln Pro Pro Arg Pro Gln Pro His Pro
   85 90 95
   Gln Pro His Pro Cys Pro Cys Gln Gln Pro His Pro Ser Pro Cys Gln
   100 105 110
   Tyr
<210> 8
   <211> 92
   <212> PRT
<400> 8
   Met Arg Val Leu Leu Val Ala Leu Ala Leu Leu Ala Leu Ala Ala Ser
   1 5 10 15
   Ala Thr Ser Thr His Thr Ser Gly Gly Cys Gly Cys Gln Pro Pro Pro
   20 25 30
   Pro Val His Leu Pro Pro Pro Val His Leu Pro Pro Pro Val His Leu
   35 40 45
   Pro Pro Pro Val His Leu Pro Pro Pro Val His Leu Pro Pro Pro Val
   50 55 60
   His Leu Pro Pro Pro Val His Val Pro Pro Pro Val His Leu Pro Pro
   65 70 75 80
   Pro Pro Cys His Tyr Pro Thr Gln Pro Pro Arg Tyr
   85 90
<210> 9
   <211> 71
   <212> PRT
<400> 9
   Met Arg Val Leu Leu Val Ala Leu Ala Leu Leu Ala Leu Ala Ala Ser
    1               5                  10                  15
   Ala Thr Ser Thr His Thr Ser Gly Gly Cys Gly Cys Gln Pro Pro Pro
               20                  25                  30
   Pro Val His Leu Pro Pro Pro Pro Cys His Tyr Pro Thr Gln Pro Pro
           35                  40                  45
   Arg Pro Gln Pro His Pro Gln Pro His Pro Cys Pro Cys Gln Gln Pro
       50                  55                  60
   His Pro Ser Pro Cys Gln Tyr
   65                  70
<210> 10
   <211> 60
   <212> PRT
<400> 10
   Met Arg Val Leu Leu Val Ala Leu Ala Leu Leu Ala Leu Ala Ala Ser
    1               5                  10                  15
   Ala Thr Ser Thr His Thr Ser Gly Gly Cys Gly Cys Gln Cys His Tyr
               20                  25                  30
   Pro Thr Gln Pro Pro Arg Pro Gln Pro His Pro Gln Pro His Pro Cys
           35                  40                  45
   Pro Cys Gln Gln Pro His Pro Ser Pro Cys Gln Tyr
       50                  55                  60